# EUROPEAN PATENT APPLICATION

(11) **EP 3 627 224 A1**
(43) Date of publication of application: **25.03.2020**
(21) Application number: 18802656.1
(22) Date of filing: 14.05.2018
(51) Int. Cl.: G03F 7/11, C08F 22/40, C08L 101/02, G03F 7/20, G03F 7/26

(54) **FILM FORMING MATERIAL FOR LITHOGRAPHY, COMPOSITION FOR FORMING FILM FOR LITHOGRAPHY, UNDERLAYER FILM FOR LITHOGRAPHY, AND PATTERN FORMING METHOD**

(30) Priority: 15.05.2017 JP 2017096264; 26.02.2018 JP 2018032460
(71) Applicant: Mitsubishi Gas Chemical Company, Inc., Tokyo 100-8324 (JP)
(72) Inventor: OKADA, Kana, Hiratsuka-shi Kanagawa 254-0016 (JP); HORIUCHI, Junya, Hiratsuka-shi Kanagawa 254-0016 (JP); MAKINOSHIMA, Takashi, Hiratsuka-shi Kanagawa 254-0016 (JP); ECHIGO, Masatoshi, Tokyo 100-8324 (JP)
(74) Representative: Müller-Boré & Partner Patentanwälte PartG mbB
(86) International application number: PCT/JP2018/018463
(87) International publication number: WO 2018/212116

(57) **Abstract**

The present invention provides a film forming material for lithography comprising a compound having a group of the following formula (0):

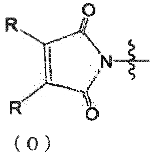

## Description

### Technical Field

The present invention relates to a film forming material for lithography, a composition for film formation for lithography containing the material, an underlayer film for lithography formed by using the composition, and a method for forming a pattern (for example, a method for forming a resist pattern or a circuit pattern) by using the composition.

### Background Art

In the production of semiconductor devices, fine processing is practiced by lithography using photoresist materials. In recent years, further miniaturization based on pattern rules has been demanded along with increase in the integration and speed of LSI. And now, lithography using light exposure, which is currently used as a general purpose technique, is approaching the limit of essential resolution derived from the wavelength of a light source.

The light source for lithography used upon forming resist patterns has been shifted to ArF excimer laser (193 nm) having a shorter wavelength from KrF excimer laser (248 nm) . However, when the miniaturization of resist patterns proceeds, the problem of resolution or the problem of collapse of resist patterns after development arises. Therefore, resists have been desired to have a thinner film. Nevertheless, if resists merely have a thinner film, it is difficult to obtain the film thicknesses of resist patterns sufficient for supporting material processing. Therefore, there has been a need for a process of preparing a resist underlayer film between a resist and a semiconductor supporting material to be processed, and imparting functions as a mask for supporting material processing to this resist underlayer film in addition to a resist pattern.

Various resist underlayer films for such a process are currently known. For example, as a material for realizing resist underlayer films for lithography having the selectivity of a dry etching rate close to that of resists, unlike conventional resist underlayer films having a fast etching rate, an underlayer film forming material for a multilayer resist process containing a resin component having at least a substituent that generates a sulfonic acid residue by eliminating a terminal group under application of predetermined energy, and a solvent has been suggested (see Patent Literature 1). Moreover, as a material for realizing resist underlayer films for lithography having the selectivity of a dry etching rate smaller than that of resists, a resist underlayer film material comprising a polymer having a specific repeat unit has been suggested (see Patent Literature 2). Furthermore, as a material for realizing resist underlayer films for lithography having the selectivity of a dry etching rate smaller than that of semiconductor supporting materials, a resist underlayer film material comprising a polymer prepared by copolymerizing a repeat unit of an acenaphthylene and a repeat unit having a substituted or unsubstituted hydroxy group has been suggested (see Patent Literature 3).

Meanwhile, as materials having high etching resistance for this kind of resist underlayer film, amorphous carbon underlayer films formed by CVD using methane gas, ethane gas, acetylene gas, or the like as a raw material are well known.

In addition, the present inventors have suggested an underlayer film forming composition for lithography containing a naphthalene formaldehyde polymer comprising a particular structural unit and an organic solvent (see Patent Literatures 4 and 5) as a material that is not only excellent in optical properties and etching resistance, but also is soluble in a solvent and applicable to a wet process.

As for methods for forming an intermediate layer used in the formation of a resist underlayer film in a three-layer process, for example, a method for forming a silicon nitride film (see Patent Literature 6) and a CVD formation method for a silicon nitride film (see Patent Literature 7) are known. Also, as intermediate layer materials for a three-layer process, materials comprising a silsesquioxane-based silicon compound are known (see Patent Literatures 8 and 9).

### Citation List

### Patent Literature

Patent Literature 1: Japanese Patent Application Laid-Open No. 2004-177668
Patent Literature 2: Japanese Patent Application Laid-Open No. 2004-271838
Patent Literature 3: Japanese Patent Application Laid-Open No. 2005-250434
Patent Literature 4: International Publication No. WO 2009/072465
Patent Literature 5: International Publication No. WO 2011/034062
Patent Literature 6: Japanese Patent Application Laid-Open No. 2002-334869
Patent Literature 7: International Publication No. WO 2004/066377
Patent Literature 8: Japanese Patent Application Laid-Open No. 2007-226170
Patent Literature 9: Japanese Patent Application Laid-Open No. 2007-226204

### Summary of Invention

### Technical Problem

As mentioned above, a large number of film forming materials for lithography have heretofore been suggested. However, none of these materials not only have high solvent solubility that permits application of a wet process such as spin coating or screen printing but also achieve all of heat resistance, etching resistance, embedding properties to a supporting material having difference in level, and film flatness at high dimensions. Thus, the development of novel materials is required.

The present invention has been made in light of the problems described above, and an object of the present invention is to provide a film forming material for lithography that is applicable to a wet process, and is useful for forming a photoresist underlayer film excellent in heat resistance, etching resistance, embedding properties to a supporting material having difference in level, and film flatness; a composition for film formation for lithography comprising the material; as well as an underlayer film for lithography and a method for forming a pattern by using the composition.

### Solution to Problem

The inventors have, as a result of devoted examinations to solve the above problems, found out that use of a compound having a specific structure can solve the above problems, and reached the present invention. More specifically, the present invention is as follows.
[1] A film forming material for lithography comprising a compound having a group of the following formula (0): 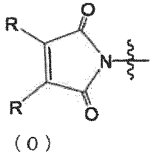 wherein each R is independently selected from the group consisting of a hydrogen atom and an alkyl group having 1 to 4 carbon atoms.
[2] The film forming material for lithography according to [1], wherein the compound having a group of the above formula (0) is at least one selected from the group consisting of a polymaleimide compound and a maleimide resin.
[3] The film forming material for lithography according to the above [1] or [2], wherein the compound having a group of the above formula (0) is at least one selected from the group consisting of a bismaleimide compound and an addition polymerization-type maleimide resin.
[4] The film forming material for lithography according to the above [3], wherein the bismaleimide compound is represented by the following formula (1): 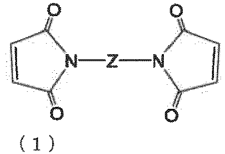 wherein Z is a divalent hydrocarbon group having 1 to 100 carbon atoms and optionally having a heteroatom.
[5A] The film forming material for lithography according to the above [3] or [4], wherein the bismaleimide compound is represented by the following formula (1A): 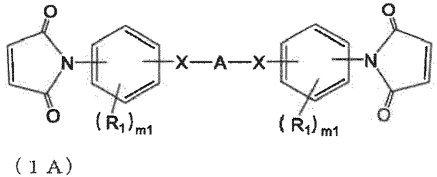 wherein
   each X is independently a single bond, -O-, -CH₂-, - C(CH₃)₂-, -CO-, -C(CF₃)₂-, -CONH- or -COO-;
   A is a single bond, an oxygen atom or a divalent hydrocarbon group having 1 to 80 carbon atoms and optionally having a heteroatom;
   each R₁ is independently a group having 0 to 30 carbon atoms and optionally having a heteroatom; and
   each m1 is independently an integer of 0 to 4.
[5B] The film forming material for lithography according to any of the above [3] to [5A], wherein the bismaleimide compound is represented by the following formula (1A): 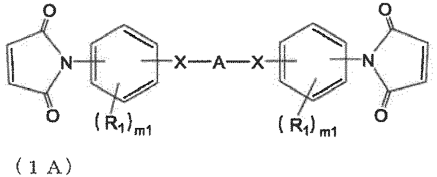 wherein
   each X is independently a single bond, -O-, -CH₂-, - C(CH₃)₂-, -CO-, -C(CF₃)₂-, -CONH- or -COO-;
   A is a divalent hydrocarbon group having 1 to 80 carbon atoms and optionally having a heteroatom;
   each R₁ is independently a group having 0 to 30 carbon atoms and optionally having a heteroatom; and
   each m1 is independently an integer of 0 to 4.
[6A] The film forming material for lithography according to any of the above [3] to [5B], wherein the bismaleimide compound is represented by the following formula (1A): 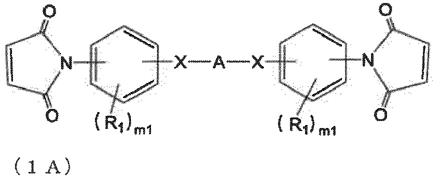 wherein
   each X is independently a single bond, -O-, -CH₂-, - C(CH₃)₂-, -CO-, -C(CF₃)₂-, -CONH- or -COO-;
   A is a single bond, an oxygen atom, -(CH₂)ₙ-, - CH₂C(CH₃)₂CH₂-, -(C(CH₃)₂)ₙ-, -(O(CH₂)ₘ₂)ₙ-, -(O(C₆H₄))ₙ- or any of the following structures: 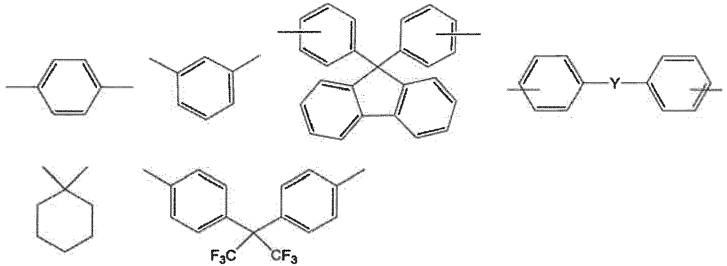
   Y is a single bond, -O-, -CH₂-, -C(CH₃)₂-, -C(CF₃)₂-, 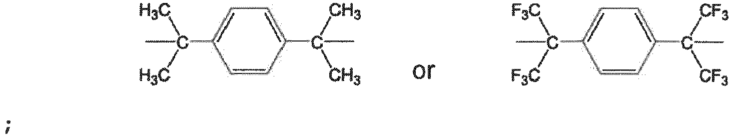
   each R₁ is independently a group having 0 to 30 carbon atoms and optionally having a heteroatom; and
   n is an integer of 0 to 20; and
   m1 and m2 are each independently an integer of 0 to 4.
[6B] The film forming material for lithography according to any of the above [3] to [6A], wherein the bismaleimide compound is represented by the following formula (1A): 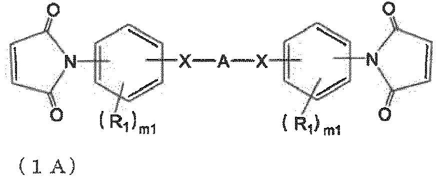 wherein
   each X is independently a single bond, -O-, -CH₂-, - C(CH₃)₂-, -CO-, -C(CF₃)₂-, -CONH- or -COO-;
   A is -(CH₂)ₙ-, -(C(CH₃)₂)ₙ-, -(O(CH₂)ₘ₂)ₙ-, -(O(C₆H₄))ₙ- or any of the following structures: 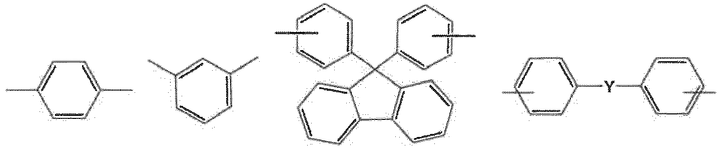
   Y is a single bond, -O-, -CH₂-, -C(CH₃)₂-, -C(CF₃)₂-, 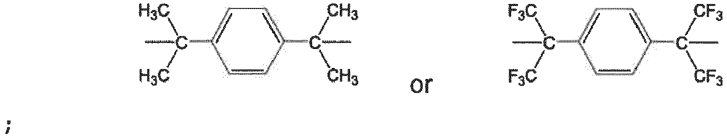
   each R₁ is independently a group having 0 to 30 carbon atoms and optionally having a heteroatom; and
   n is an integer of 0 to 20; and
   m1 and m2 are each independently an integer of 0 to 4.
[6-1A] The film forming material for lithography according to any of the above [3] to [6B], wherein the bismaleimide compound is represented by the following formula (1A): 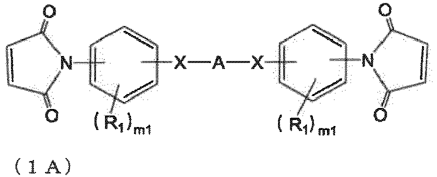 wherein
   each X is independently a single bond, -O-, -CO- or -COO-;
   A is a single bond, an oxygen atom, -(CH₂)ₙ₁-, - CH₂C(CH₃)₂CH₂-, -(O(CH₂)ₙ₂)ₙ₃- or the following structures: 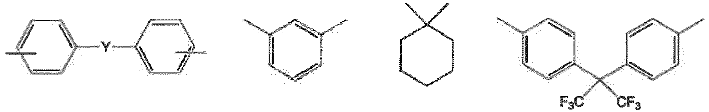
   n1 is an integer of 1 to 10;
   n2 is an integer of 1 to 4;
   n3 is an integer of 1 to 20;
   Y is -C(CH₃)₂- or -C(CF₃)₂-;
   each R₁ is independently an alkyl group; and
   each m1 is independently an integer of 0 to 4.
[6-1B] The film forming material for lithography according to any of the above [3] to [6-1A], wherein the bismaleimide compound is represented by the following formula (1A): 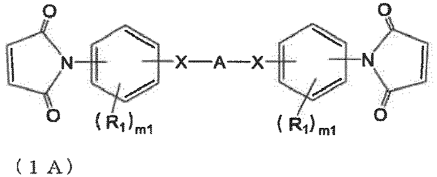 wherein
   each X is independently a single bond, -O-, -CO- or -COO-;
   A is -(CH₂)ₙ₁-, -(O(CH₂)ₙ₂)ₙ₃- or the following structures: 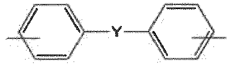
   n1 is an integer of 1 to 10;
   n2 is an integer of 1 to 4;
   n3 is an integer of 1 to 20;
   Y is -C(CH₃)₂- or -C(CF₃)₂-;
   each R₁ is independently an alkyl group; and
   each m1 is independently an integer of 0 to 4.
[6-2] The film forming material for lithography according to the above [6-1B], wherein
   X is a single bond;
   A is -(CH₂)ₙ₁-;
   n1 is an integer of 1 to 10;
   each R₁ is independently an alkyl group; and
   each m1 is independently an integer of 0 to 4.
[6-3] The film forming material for lithography according to the above [6-2], wherein n1 is an integer of 1 to 6.
[6-4] The film forming material for lithography according to the above [6-2], wherein n1 is an integer of 1 to 3.
[6-5] The film forming material for lithography according to the above [6-1B], wherein
   each X is independently -CO- or -COO-;
   A is -(O(CH₂)ₙ₂)ₙ₃-;
   n2 is an integer of 1 to 4;
   n3 is an integer of 1 to 20;
   each R₁ is independently an alkyl group; and
   each m1 is independently an integer of 0 to 4.
[6-6] The film forming material for lithography according to the above [6-5], wherein -X-A-X- is -CO-(O(CH₂)ₙ₂)ₙ₃-COO-.
[6-7] The film forming material for lithography according to the above [6-1B], wherein
   X is -O-;
   A is the following structure: 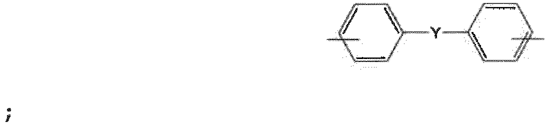 Y is -C(CH₃)₂- or -C(CF₃)₂-;
   each R₁ is independently an alkyl group; and
   each m1 is independently an integer of 0 to 4.
[6-8] The film forming material for lithography according to the above [6-7], wherein
   A is the following structure: 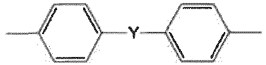
[6-9] The film forming material for lithography according to any of the above [5A] to [6-8], wherein each R₁ is independently an alkyl group having 1 to 6 carbon atoms.
[6-10] The film forming material for lithography according to any of the above [5A] to [6-8], wherein each R₁ is independently an alkyl group having 1 to 3 carbon atoms.
[7] The film forming material for lithography according to any of the above [3] to [6-10], wherein the addition polymerization-type maleimide resin is represented by the following formula (2): 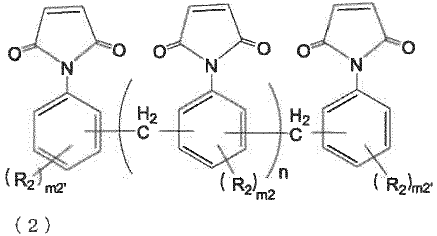 wherein
   each R₂ is independently a group having 0 to 10 carbon atoms and optionally having a heteroatom;
   each m2 is independently an integer of 0 to 3;
   each m2' is independently an integer of 0 to 4; and
   n is an integer of 1 to 4,
   or the following formula (3): 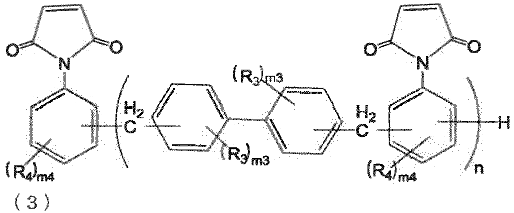 wherein
   R₃ and R₄ are each independently a group having 0 to 10 carbon atoms and optionally having a heteroatom;
   each m3 is independently an integer of 0 to 4;
   each m4 is independently an integer of 0 to 4; and
   n is an integer of 1 to 4.
[7-1] The film forming material for lithography according to the above [7], wherein R₂, or R₃ and R₄ are alkyl groups.
[7-2] The film forming material for lithography according to any of the above [4] to [7-1], wherein the heteroatom is selected from the group consisting of oxygen, fluorine, and silicon.
[7-3] The film forming material for lithography according to any of the above [4] to [7-2], wherein the heteroatom is oxygen.
[8] The film forming material for lithography according to any of the above [1] to [7-3], further comprising a crosslinking agent.
[9] The film forming material for lithography according to the above [8], wherein the crosslinking agent is at least one selected from the group consisting of a phenol compound, an epoxy compound, a cyanate compound, an amino compound, a benzoxazine compound, a melamine compound, a guanamine compound, a glycoluril compound, a urea compound, an isocyanate compound and an azide compound.
[10] The film forming material for lithography according to the above [8] or [9], wherein the crosslinking agent has at least one allyl group.
[11] The film forming material for lithography according to any of the above [8] to [10], wherein a content ratio of the crosslinking agent is 0.1 to 100 parts by mass based on 100 parts by mass of a total mass of the bismaleimide compound and the addition polymerization-type maleimide resin.
[12] The film forming material for lithography according to any of the above [1] to [11], further comprising a crosslinking promoting agent.
[13] The film forming material for lithography according to the above [12], wherein the crosslinking promoting agent is at least one selected from the group consisting of an amine, an imidazole, an organic phosphine and a Lewis acid.
[14] The film forming material for lithography according to the above [12] or [13], wherein a content ratio of the crosslinking promoting agent is 0.1 to 5 parts by mass based on 100 parts by mass of a total mass of the bismaleimide compound and the addition polymerization-type maleimide resin.
[15] The film forming material for lithography according to any of the above [1] to [14], further comprising a radical polymerization initiator.
[16] The film forming material for lithography according to the above [15], wherein the radical polymerization initiator is at least one selected from the group consisting of a ketone-based photopolymerization initiator, an organic peroxide-based polymerization initiator and an azo-based polymerization initiator.
[17] The film forming material for lithography according to the above [15] or [16], wherein a content ratio of the radical polymerization initiator is 0.05 to 25 parts by mass based on 100 parts by mass of a total mass of the bismaleimide compound and the addition polymerization-type maleimide resin.
[18] A composition for film formation for lithography comprising the film forming material for lithography according to any of the above [1] to [17] and a solvent.
[19] The composition for film formation for lithography according to the above [18], further comprising an acid generating agent.
[20] The composition for film formation for lithography according to the above [18] or [19], wherein the film for lithography is an underlayer film for lithography.
[21] An underlayer film for lithography formed by using the composition for film formation for lithography according to the above [20].
[22] A method for forming a resist pattern, comprising the steps of:
   forming an underlayer film on a supporting material by using the composition for film formation for lithography according to the above [20];
   forming at least one photoresist layer on the underlayer film; and
   irradiating a predetermined region of the photoresist layer with radiation for development.
[23] A method for forming a circuit pattern, comprising the steps of:
   forming an underlayer film on a supporting material by using the composition for film formation for lithography according to the above [20];
   forming an intermediate layer film on the underlayer film by using a resist intermediate layer film material having a silicon atom;
   forming at least one photoresist layer on the intermediate layer film;
   irradiating a predetermined region of the photoresist layer with radiation for development, thereby forming a resist pattern;
   etching the intermediate layer film with the resist pattern as a mask;
   etching the underlayer film with the obtained intermediate layer film pattern as an etching mask; and
   etching the supporting material with the obtained underlayer film pattern as an etching mask, thereby forming a pattern on the supporting material.
[24] A purification method comprising the steps of:
   obtaining an organic phase by dissolving the film forming material for lithography according to any of the above [1] to [17] in a solvent; and
   extracting impurities in the film forming material for lithography by bringing the organic phase into contact with an acidic aqueous solution (a first extraction step), wherein
   the solvent used in the step of obtaining the organic phase comprises a solvent that is incompatible with water.
[25] The purification method according to the above [24], wherein the acidic aqueous solution is an aqueous mineral acid solution or an aqueous organic acid solution;
   the aqueous mineral acid solution comprises one or more selected from the group consisting of hydrochloric acid, sulfuric acid, nitric acid and phosphoric acid; and
   the aqueous organic acid solution comprises one or more selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid and trifluoroacetic acid.
[26] The purification method according to the above [24] or [25], wherein the solvent that is incompatible with water is one or more solvents selected from the group consisting of toluene, 2-heptanone, cyclohexanone, cyclopentanone, methyl isobutyl ketone, propylene glycol monomethyl ether acetate and ethyl acetate.
[27] The purification method according to any of the above [24] to [26], further comprising the step of extracting impurities in the film forming material for lithography by bringing the organic phase into contact with water after the first extraction step (a second extraction step).

### Advantageous Effects of Invention

The present invention can provide a film forming material for lithography that is applicable to a wet process, and is useful for forming a photoresist underlayer film excellent in heat resistance, etching resistance, embedding properties to a supporting material having difference in level, and film flatness; a composition for film formation for lithography comprising the material; as well as an underlayer film for lithography and a method for forming a pattern by using the composition.

### Description of Embodiments

Hereinafter, embodiments of the present invention will be described. The embodiments described below are given merely for illustrating the present invention. The present invention is not limited only by these embodiments.

### [Film forming material for lithography]

A film forming material for lithography, which is one of the embodiments of the present invention, comprises a compound having a group of the following formula (0): 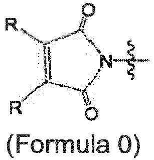 wherein each R is independently selected from the group consisting of a hydrogen atom and an alkyl group having 1 to 4 carbon atoms.
Hereinafter, the compound may be referred to as a "maleimide compound." The maleimide compound can be obtained by conducting a ring closure reaction with dehydration between, for example, a compound having one or more primary amino groups in the molecule and maleic anhydride. In addition, the maleimide compound includes, for example, those similarly subjected to methylmaleimidization by using citraconic anhydride or the like instead of maleic anhydride. Examples of the maleimide compound may include, for example, a polymaleimide compound and a maleimide resin.

The content of the maleimide compound in the film forming material for lithography of the present embodiment is preferably 0.1 to 100% by mass, more preferably 0.5 to 100% by mass, and further preferably 1 to 100% by mass. In addition, from the viewpoint of heat resistance and etching resistance, the content is preferably 51 to 100% by mass, more preferably 60 to 100% by mass, further preferably 70 to 100% by mass, and particularly preferably 80 to 100% by mass.

The maleimide compound according to the present embodiment can be used in combination with a conventional, underlayer film forming composition in order to improve heat resistance of the conventional, underlayer film forming composition. In that case, the content of the maleimide compound in the underlayer film forming composition (excluding a solvent) is preferably 1 to 50% by mass and more preferably 1 to 30% by mass.

Examples of the conventional, underlayer film forming composition may include, but are not limited to, those descried in International Publication No. WO 2013/024779, for example.

The maleimide compound in the film forming material for lithography of the present embodiment is characterized by having a function other than those as an acid generating agent or a basic compound for film formation for lithography.

For the polymaleimide compound and the maleimide resin used in the film forming material for lithography of the present embodiment, a bismaleimide compound and an addition polymerization-type maleimide resin are preferable from the viewpoint of the availability of raw materials and mass production.

### <Bismaleimide compound>

The bismaleimide compound is preferably a compound represented by the following formula (1): 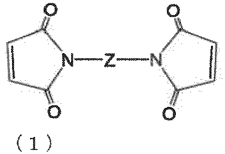 wherein Z is a divalent hydrocarbon group having 1 to 100 carbon atoms and optionally containing a heteroatom. The number of carbon atoms in the hydrocarbon group may be 1 to 80, 1 to 60, 1 to 40, 1 to 20, or the like. Examples of the heteroatom may include oxygen, nitrogen, sulfur, fluorine, silicon, and the like.

The bismaleimide compound is more preferably a compound represented by the following formula (1A): 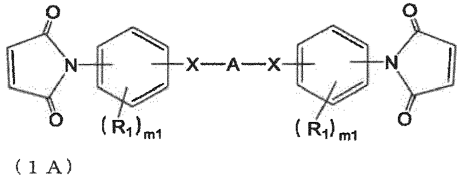 wherein
each X is independently a single bond, -O-, -CH₂-, - C(CH₃)₂-, -CO-, -C(CF₃)₂-, -CONH- or -COO-;
A is a single bond, an oxygen atom or a divalent hydrocarbon group having 1 to 80 carbon atoms and optionally containing a heteroatom (for example, oxygen, nitrogen, sulfur, fluorine);
each R₁ is independently a group having 0 to 30 carbon atoms and optionally containing a heteroatom (for example, oxygen, nitrogen, sulfur, fluorine, chlorine, bromine, iodine); and
each m1 is independently an integer of 0 to 4.

More preferably, from the viewpoint of improvement in heat resistance and etching resistance, in formula (1A),
each X is independently a single bond, -O-, -CH₂-, - C(CH₃)₂-, -CO-, -C(CF₃)₂-, -CONH- or -COO-;
A is a single bond, an oxygen atom, -(CH₂)ₙ-, - CH₂C(CH₃)₂CH₂-, -(C(CH₃)₂)ₙ-, -(O(CH₂)ₘ₂)ₙ-, -(O(C₆H₄))ₙ- or any of the following structures: 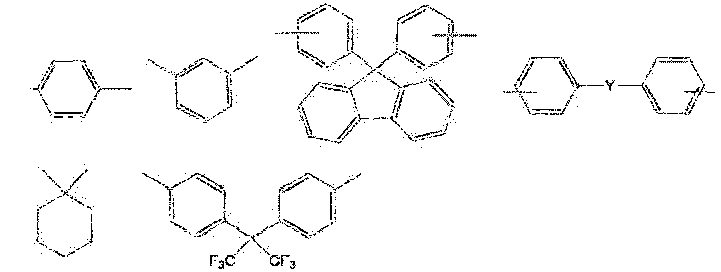 Y is a single bond, -O-, -CH₂-, -C(CH₃)₂-, -C(CF₃)₂-, 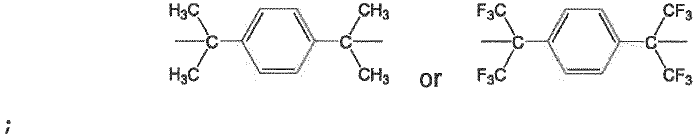 each R₁ is independently a group having 0 to 30 carbon atoms and optionally containing a heteroatom (for example, oxygen, nitrogen, sulfur, fluorine, chlorine, bromine, iodine); and
n is an integer of 0 to 20; and
m1 and m2 are each independently an integer of 0 to 4.

X is preferably a single bond from the viewpoint of heat resistance, and is preferably -COO- from the viewpoint of solubility.

Y is preferably a single bond from the viewpoint of improvement in heat resistance.

R₁ is preferably a group having 0 to 20 or 0 to 10 carbon atoms and optionally containing a heteroatom (for example, oxygen, nitrogen, sulfur, fluorine, chlorine, bromine, iodine). R₁ is preferably a hydrocarbon group from the viewpoint of improvement in solubility in an organic solvent. For example, examples of R₁ include an alkyl group (for example, an alkyl group having 1 to 6 or 1 to 3 carbon atoms) and the like, and specific examples include a methyl group, an ethyl group and the like.

m1 is preferably an integer of 0 to 2, and is more preferably 1 or 2 from the viewpoint of the availability of raw materials and improved solubility.

m2 is preferably an integer of 2 to 4.

n is preferably an integer of 0 to 2, and is more preferably an integer of 1 to 2 from the viewpoint of improvement in heat resistance.

In one embodiment of the compound represented by formula (1A),
each X is independently a single bond, -O-, -CO- or -COO-;
A is a single bond, an oxygen atom, -(CH₂)ₙ₁-, - CH₂C(CH₃)₂CH₂-, -(O(CH₂)ₙ₂)ₙ₃- or the following structures: 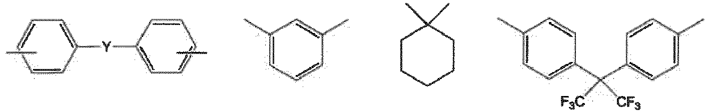 n1 is an integer of 1 to 10;
n2 is an integer of 1 to 4;
n3 is an integer of 1 to 20;
Y is -C(CH₃)₂- or -C(CF₃)₂-;
each R₁ is independently an alkyl group (for example, an alkyl group having 1 to 6 or 1 to 3 carbon atoms); and
each m1 is independently an integer of 0 to 4.

In one embodiment of the compound represented by formula (1A),
X is a single bond;
A is -(CH₂)ₙ₁-;
n1 is an integer of 1 to 10;
each R₁ is independently an alkyl group (for example, an alkyl group having 1 to 6 or 1 to 3 carbon atoms); and
each m1 is independently an integer of 0 to 4.

Here, n1 is preferably 1 to 6 or 1 to 3.

In one embodiment of the compound represented by formula (1A),
each X is independently -CO- or -COO-;
A is -(O(CH₂)ₙ₂)ₙ₃-;
n2 is an integer of 1 to 4;
n3 is an integer of 1 to 20;
each R₁ is independently an alkyl group (for example, an alkyl group having 1 to 6 or 1 to 3 carbon atoms); and
each m1 is independently an integer of 0 to 4.

Here, -X-A-X- is preferably -CO-(O(CH₂)ₙ₂)ₙ₃-COO-.

In one embodiment of the compound represented by formula (1A),
X is -O-;
A is the following structure: 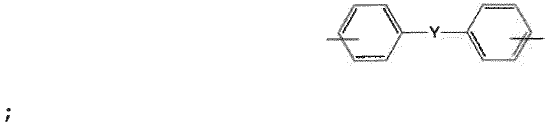 Y is -C(CH₃)₂- or -C(CF₃)₂-;
each R₁ is independently an alkyl group (for example, an alkyl group having 1 to 6 or 1 to 3 carbon atoms); and
each m1 is independently an integer of 0 to 4.

Here, A is preferably the following structure:

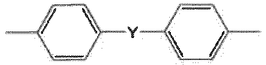

The bismaleimide compound is preferably a compound represented by the following formula (1B): 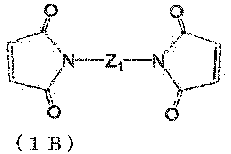 wherein Z1 is a linear, branched or cyclic, divalent hydrocarbon group having 1 to 100 carbon atoms and optionally containing a heteroatom. Examples of the heteroatom may include oxygen, nitrogen, sulfur, fluorine, silicon, and the like.

### <Addition polymerization-type maleimide resin>

The addition polymerization-type maleimide resin is preferably a resin represented by the following formula (2) or the following formula (3), from the viewpoint of improvement in etching resistance.

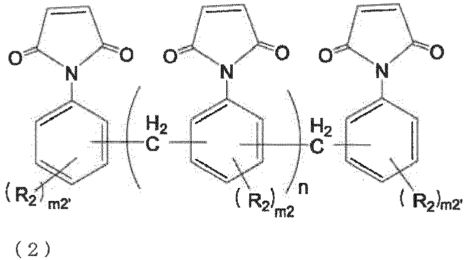

In the above formula (2), each R₂ is independently a group having 0 to 10 carbon atoms and optionally containing a heteroatom (for example, oxygen, nitrogen, sulfur, fluorine, chlorine, bromine, iodine). In addition, R₂ is preferably a hydrocarbon group from the viewpoint of improvement in solubility in an organic solvent. For example, examples of R₂ include an alkyl group (for example, an alkyl group having 1 to 6 or 1 to 3 carbon atoms) and the like, and specific examples include a methyl group, an ethyl group and the like.

Each m2 is independently an integer of 0 to 3. In addition, m2 is preferably 0 or 1, and is more preferably 0 from the viewpoint of the availability of raw materials.

Each m2' is independently an integer of 0 to 4. In addition, m2' is preferably 0 or 1, and is more preferably 0 from the viewpoint of the availability of raw materials.

n is an integer of 0 to 4. In addition, n is preferably an integer of 1 to 4 or 0 to 2, and is more preferably an integer of 1 to 2 from the viewpoint of improvement in heat resistance.

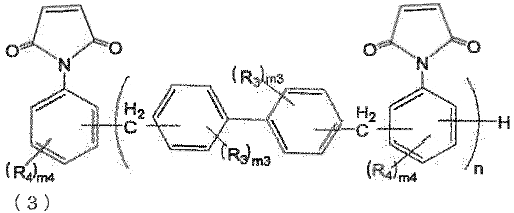

In the above formula (3), R₃ and R₄ are each independently a group having 0 to 10 carbon atoms and optionally containing a heteroatom (for example, oxygen, nitrogen, sulfur, fluorine, chlorine, bromine, iodine). In addition, R₃ and R₄ are preferably hydrocarbon groups from the viewpoint of improvement in solubility in an organic solvent. For example, examples of R₃ and R₄ include an alkyl group (for example, an alkyl group having 1 to 6 or 1 to 3 carbon atoms) and the like, and specific examples include a methyl group, an ethyl group and the like.

Each m3 is independently an integer of 0 to 4. In addition, m3 is preferably an integer of 0 to 2, and is more preferably 0 from the viewpoint of the availability of raw materials.

Each m4 is independently an integer of 0 to 4. In addition, m4 is preferably an integer of 0 to 2, and is more preferably 0 from the viewpoint of the availability of raw materials.

n is an integer of 0 to 4. In addition, n is preferably an integer of 1 to 4 or 0 to 2, and is more preferably an integer of 1 to 2 from the viewpoint of the availability of raw materials.

The film forming material for lithography of the present embodiment is applicable to a wet process. In addition, the film forming material for lithography of the present embodiment has an aromatic structure and also has a rigid maleimide skeleton, and therefore, when it is baked at a high temperature, its maleimide group undergoes a crosslinking reaction even on its own, thereby expressing high heat resistance. As a result, deterioration of the film upon baking at a high temperature is suppressed and an underlayer film excellent in etching resistance to oxygen plasma etching and the like can be formed. Furthermore, even though the film forming material for lithography of the present embodiment has an aromatic structure, its solubility in an organic solvent is high and its solubility in a safe solvent is high. Furthermore, an underlayer film for lithography composed of the composition for film formation for lithography of the present embodiment, which will be mentioned later, is not only excellent in embedding properties to a supporting material having difference in level and film flatness, thereby having a good stability of the product quality, but also excellent in adhesiveness to a resist layer or a resist intermediate layer film material, and thus, an excellent resist pattern can be obtained.

Specific examples of the bismaleimide compound used in the present embodiment include phenylene skeleton containing bismaleimides such as m-phenylene bismaleimide, 4-methyl-1,3-phenylene bismaleimide, 4,4-diphenylmethane bismaleimide, 4,4'-diphenylsulfone bismaleimide, 1,3-bis(3-meleimidephenoxy)benzene, 1,3-bis(4-meleimidephenoxy)benzene, 1,4-bis(3-meleimidephenoxy)benzene and 1,4-bis(4-meleimidephenoxy)benzene; diphenylalkane skeleton containing bismaleimides such as bis(3-ethyl-5-methyl-4-meleimidephenyl)methane, 1,1-bis(3-ethyl-5-methyl-4-meleimidephenyl)ethane, 2,2-bis(3-ethyl-5-methyl-4-meleimidephenyl)propane, N,N'-4,4'-[3,3'-dimethyl-diphenylmethane]bismaleimide, N,N'-4,4'-[3,3'-dimethyl-1,1-diphenylethane]bismaleimide, N,N'-4,4'-[3,3'-dimethyl-1,1-diphenylpropane]bismaleimide, N,N'-4,4'-[3,3'-diethyl-diphenylmethane]bismaleimide, N,N'-4,4'-[3,3'-di-n-propyl-diphenylmethane]bismaleimide and N,N'-4,4'-[3,3'-di-n-butyl-diphenylmethane]bismaleimide; biphenyl skeleton containing bismaleimides such as N,N'-4,4'-[3,3'-dimethyl-biphenylene]bismaleimide and N,N'-4,4'-[3,3'-diethyl-biphenylene]bismaleimide; aliphatic skeleton bismaleimides such as 1,6-hexane bismaleimide, 1,6-bismaleimide-(2,2,4-trimethyl)hexane, 1,3-dimethylenecyclohexane bismaleimide and 1,4-dimethylenecyclohexane bismaleimide; bismaleimide compounds composed of diamino siloxane such as 1,3-bis(3-aminopropyl)-1,1,2,2-tetramethyl disiloxane, 1,3-bis(3-aminobutyl)-1,1,2,2-tetramethyl disiloxane, bis(4-aminophenoxy)dimethyl silane, 1,3-bis(4-aminophenoxy)tetramethyl disiloxane, 1,1,3,3-tetramethyl-1,3-bis(4-aminophenyl)disiloxane, 1,1,3,3-tetraphenoxy-1,3-bis(2-aminoethyl)disiloxane, 1,1,3,3-tetraphenyl-1,3-bis(2-aminoethyl)disiloxane, 1,1,3,3-tetraphenyl-1,3-bis(3-aminopropyl)disiloxane, 1,1,3,3-tetramethyl-1,3-bis(2-aminoethyl)disiloxane, 1,1,3,3-tetramethyl-1,3-bis(3-aminopropyl)disiloxane, 1,1,3,3-tetramethyl-1,3-bis(4-aminobutyl)disiloxane, 1,3-dimethyl-1,3-dimethoxy-1,3-bis(4-aminobutyl)disiloxane, 1,1,3,3,5,5-hexamethyl-1,5-bis(4-aminophenyl)trisiloxane, 1,1,5,5-tetraphenyl-3,3-dimethyl-1,5-bis(3-aminopropyl)trisiloxane, 1,1,5,5-tetraphenyl-3,3-dimethoxy-1,5-bis(4-aminobutyl)trisiloxane, 1,1,5,5-tetraphenyl-3,3-dimethoxy-1,5-bis(5-aminopentyl)trisiloxane, 1,1,5,5-tetramethyl-3,3-dimethoxy-1,5-bis(2-aminoethyl)trisiloxane, 1,1,5,5-tetramethyl-3,3-dimethoxy-1,5-bis(4-aminobutyl)trisiloxane, 1,1,5,5-tetramethyl-3,3-dimethoxy-1,5-bis(5-aminopentyl)trisiloxane, 1,1,3,3,5,5-hexamethyl-1,5-bis(3-aminopropyl)trisiloxane, 1,1,3,3,5,5-hexaethyl-1,5-bis(3-aminopropyl)trisiloxane and 1,1,3,3,5,5-hexapropyl-1,5-bis(3-aminopropyl)trisiloxane; and the like.

Among bismaleimide compounds, bis(3-ethyl-5-methyl-4-meleimidephenyl)methane, N,N'-4,4'-[3,3'-dimethyl-diphenylmethane]bismaleimide and N,N'-4,4'-[3,3'-diethyl-diphenylmethane]bismaleimide are particularly preferable because they are excellent in solvent solubility and heat resistance.

Examples of the addition polymerization-type maleimide resin used in the present embodiment include, for example, Bismaleimide M-20 (manufactured by Mitsui Toatsu Chemicals, trade name), BMI-2300 (manufactured by Daiwa Kasei Industry Co., Ltd., trade name), BMI-3200 (manufactured by Daiwa Kasei Industry Co., Ltd., trade name), MIR-3000 (manufactured by Nippon Kayaku Co., Ltd., product name) and the like. Among them, BMI-2300 is particularly preferable because it is excellent is solubility and heat resistance.

### <Crosslinking agent>

The film forming material for lithography of the present embodiment may comprise a crosslinking agent, if required, in addition to a bismaleimide compound and/or an addition polymerization-type maleimide resin from the viewpoint of lowering the curing temperature, suppressing intermixing and the like.

The crosslinking agent is not particularly limited as long as it undergoes a crosslinking reaction with maleimide, and any of known crosslinking systems can be applied, but specific examples of the crosslinking agent that may be used in the present embodiment include, but are not particularly limited to, phenol compounds, epoxy compounds, cyanate compounds, amino compounds, benzoxazine compounds, acrylate compounds, melamine compounds, guanamine compounds, glycoluril compounds, urea compounds, isocyanate compounds, azide compounds and the like. These crosslinking agents can be used alone as one kind or can be used in combination of two or more kinds. Among them, benzoxazine compounds, epoxy compounds or cyanate compounds are preferable, and benzoxazine compounds are more preferable from the viewpoint of improvement in etching resistance.

In a crosslinking reaction between maleimide and the crosslinking agent, for example, an active group these crosslinking agents have (a phenolic hydroxy group, an epoxy group, a cyanate group, an amino group, or a phenolic hydroxy group formed by ring opening of the alicyclic site of benzoxazine) undergoes an addition reaction with a carbon-carbon double bond that constitutes a maleimide group to form crosslinkage. Besides, two carbon-carbon double bonds that the bismaleimide compound of the present embodiment has are polymerized to form crosslinkage.

As the above phenol compound, a known compound can be used. Examples of phenols include phenol as well as alkylphenols such as cresols and xylenols, polyhydric phenols such as hydroquinone, polycyclic phenols such as naphthols and naphthalenediols, bisphenols such as bisphenol A and bisphenol F, and polyfunctional phenol compounds such as phenol novolac and phenol aralkyl resins. Preferably, an aralkyl-based phenol resin is desirable from the viewpoint of heat resistance and solubility.

As the above epoxy compound, a known compound can be used and is selected from among compounds having two or more epoxy groups in one molecule. Examples thereof include, but are not particularly limited to, epoxidation products of dihydric phenols such as bisphenol A, bisphenol F, 3,3',5,5'-tetramethyl-bisphenol F, bisphenol S, fluorene bisphenol, 2,2'-biphenol, 3,3',5,5'-tetramethyl-4,4'-dihydroxybiphenol, resorcin, and naphthalenediols, epoxidation products of trihydric or higher phenols such as tris-(4-hydroxyphenyl)methane, 1,1,2,2-tetrakis(4-hydroxyphenyl)ethane, tris(2,3-epoxypropyl) isocyanurate, trimethylolmethane triglycidyl ether, trimethylolpropane triglycidyl ether, triethylolethane triglycidyl ether, phenol novolac, and o-cresol novolac, epoxidation products of co-condensed resins of dicyclopentadiene and phenols, epoxidation products of phenol aralkyl resins synthesized from phenols and paraxylylene dichloride, epoxidation products of biphenyl aralkyl-based phenolic resins synthesized from phenols and bischloromethylbiphenyl, and epoxidation products of naphthol aralkyl resins synthesized from naphthols and paraxylylene dichloride. These epoxy resins may be used alone or in combination of two or more kinds. An epoxy resin that is in a solid state at normal temperature, such as an epoxy resin obtained from a phenol aralkyl resin or a biphenyl aralkyl resin is preferable from the viewpoint of heat resistance and solubility.

The above cyanate compound is not particularly limited as long as the compound has two or more cyanate groups in one molecule, and a known compound can be used. For example, examples thereof include those described in WO 2011108524, but preferable examples of the cyanate compound in the present embodiment include cyanate compounds having a structure where hydroxy groups of a compound having two or more hydroxy groups in one molecule are replaced with cyanate groups. Also, the cyanate compound preferably has an aromatic group, and a structure where a cyanate group is directly bonded to an aromatic group can be preferably used. Examples of such a cyanate compound include cyanate compounds having a structure where hydroxy groups of bisphenol A, bisphenol F, bisphenol M, bisphenol P, bisphenol E, a phenol novolac resin, a cresol novolac resin, a dicyclopentadiene novolac resin, tetramethylbisphenol F, a bisphenol A novolac resin, brominated bisphenol A, a brominated phenol novolac resin, trifunctional phenol, tetrafunctional phenol, naphthalene-based phenol, biphenyl-based phenol, a phenol aralkyl resin, a biphenyl aralkyl resin, a naphthol aralkyl resin, a dicyclopentadiene aralkyl resin, alicyclic phenol, phosphorus-containing phenol, or the like are replaced with cyanate groups. These cyanate compounds may be used alone or in arbitrary combination of two or more kinds. Also, the above cyanate compound may be in any form of a monomer, an oligomer and a resin.

Examples of the above amino compound include m-phenylenediamine, p-phenylenediamine, 4,4'-diaminodiphenylmethane, 4,4'-diaminodiphenylpropane, 4,4'-diaminodiphenyl ether, 3,4'-diaminodiphenyl ether, 3,3'-diaminodiphenyl ether, 4,4'-diaminodiphenylsulfone, 3,4'-diaminodiphenylsulfone, 3,3'-diaminodiphenylsulfone, 4,4'-diaminodiphenyl sulfide, 3,4'-diaminodiphenyl sulfide, 3,3'-diaminodiphenyl sulfide, 1,4-bis(4-aminophenoxy)benzene, 1,3-bis(4-aminophenoxy)benzene, 1,4-bis(3-aminophenoxy)benzene, 1,3-bis(3-aminophenoxy)benzene, bis[4-(4-aminophenoxy)phenyl]sulfone, 2,2-bis[4-(4-aminophenoxy)phenyl]propane, 2,2-bis[4-(3-aminophenoxy)phenyl]propane, 4,4'-bis(4-aminophenoxy)biphenyl, 4,4'-bis(3-aminophenoxy)biphenyl, bis[4-(4-aminophenoxy)phenyl] ether, bis[4-(3-aminophenoxy)phenyl] ether, 9,9-bis(4-aminophenyl)fluorene, 9,9-bis(4-amino-3-chlorophenyl)fluorene, 9,9-bis(4-amino-3-fluorophenyl)fluorene, O-tolidine, m-tolidine, 4,4'-diaminobenzanilide, 2,2'-bis(trifluoromethyl)-4,4'-diaminobiphenyl, 4-aminophenyl-4-aminobenzoate, and 2-(4-aminophenyl)-6-aminobenzoxazole. Among them, examples thereof include aromatic amines such as 4,4'-diaminodiphenylmethane, 4,4'-diaminodiphenylpropane, 4,4'-diaminodiphenyl ether, 3,4'-diaminodiphenyl ether, 3,3'-diaminodiphenyl ether, 4,4'-diaminodiphenylsulfone, 3,3'-diaminodiphenylsulfone, 1,4-bis(4-aminophenoxy)benzene, 1,3-bis(4-aminophenoxy)benzene, 1,4-bis(3-aminophenoxy)benzene, 1,3-bis(3-aminophenoxy)benzene, bis[4-(4-aminophenoxy)phenyl]sulfone, 2,2-bis[4-(4-aminophenoxy)phenyl]propane, 2,2-bis[4-(3-aminophenoxy)phenyl]propane, 4,4'-bis(4-aminophenoxy)biphenyl, 4,4'-bis(3-aminophenoxy)biphenyl, bis[4-(4-aminophenoxy)phenyl] ether, and bis[4-(3-aminophenoxy)phenyl] ether, alicyclic amines such as diaminocyclohexane, diaminodicyclohexylmethane, dimethyl-diaminodicyclohexylmethane, tetramethyl-diaminodicyclohexylmethane, diaminodicyclohexylpropane, diaminobicyclo[2.2.1]heptane, bis(aminomethyl)-bicyclo[2.2.1]heptane, 3(4),8(9)-bis(aminomethyl)tricyclo[5.2.1.02,6]decane, 1,3-bisaminomethylcyclohexane, and isophoronediamine, and aliphatic amines such as ethylenediamine, hexamethylenediamine, octamethylenediamine, decamethylenediamine, diethylenetriamine, and triethylenetetramine.

The structure of oxazine of the above benzoxazine compound is not particularly limited, and examples thereof include a structure of oxazine having an aromatic group including a condensed polycyclic aromatic group, such as benzoxazine and naphthoxazine.

Examples of the benzoxazine compound include, for example, compounds represented by the following general formulas (a) to (f). Note that, in the general formulas described below, a bond displayed toward the center of a ring indicates a bond to any carbon that constitutes the ring and to which a substituent can be bonded.

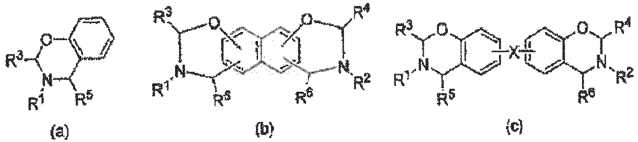

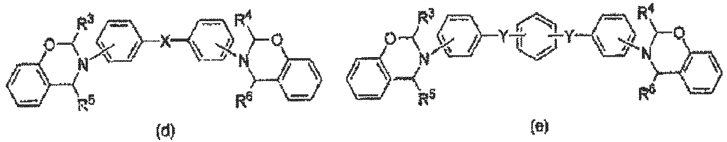

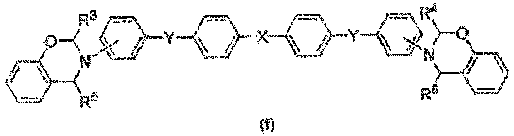

In the general formulas (a) to (c), R1 and R2 independently represent an organic group having 1 to 30 carbon atoms. In addition, in the general formulas (a) to (f), R3 to R6 independently represent hydrogen or a hydrocarbon group having 1 to 6 carbon atoms. Moreover, in the above general formulas (c), (d) and (f), X independently represents a single bond, -O-, -S-, -S-S-, -SO₂-, -CO-, -CONH-, -NHCO-, -C(CH₃)₂-, -C(CF₃)₂-, - (CH₂)m-, -O-(CH₂)m-O- or -S-(CH₂)m-S-. Here, m is an integer of 1 to 6. In addition, in the general formulas (e) and (f), Y independently represents a single bond, - O-, -S-, -CO-, -C(CH₃)₂-, -C(CF₃)₂- or alkylene having 1 to 3 carbon atoms.

Moreover, the benzoxazine compound includes an oligomer or polymer having an oxazine structure as a side chain, and an oligomer or polymer having a benzoxazine structure in the main chain.

The benzoxazine compound can be produced in a similar method as a method described in International Publication No. WO 2004/009708, Japanese Patent Application Laid-Open No. 11-12258 or Japanese Patent Application Laid-Open No. 2004-352670.

Specific examples of the melamine compounds include hexamethylolmelamine, hexamethoxymethylmelamine, a compound in which 1 to 6 methylol groups of hexamethylolmelamine are methoxymethylated or a mixture thereof, hexamethoxyethylmelamine, hexaacyloxymethylmelamine, and a compound in which 1 to 6 methylol groups of hexamethylolmelamine are acyloxymethylated or a mixture thereof.

Specific examples of the guanamine compounds include tetramethylolguanamine, tetramethoxymethylguanamine, a compound in which 1 to 4 methylol groups of tetramethylolguanamine are methoxymethylated or a mixture thereof, tetramethoxyethylguanamine, tetraacyloxyguanamine, and a compound in which 1 to 4 methylol groups of tetramethylolguanamine are acyloxymethylated or a mixture thereof.

Specific examples of the glycoluril compounds include tetramethylolglycoluril, tetramethoxyglycoluril, tetramethoxymethylglycoluril, a compound in which 1 to 4 methylol groups of tetramethylolglycoluril are methoxymethylated or a mixture thereof, and a compound in which 1 to 4 methylol groups of tetramethylolglycoluril are acyloxymethylated or a mixture thereof.

Specific examples of the urea compounds include tetramethylolurea, tetramethoxymethylurea, a compound in which 1 to 4 methylol groups of tetramethylolurea are methoxymethylated or a mixture thereof, and tetramethoxyethylurea.

In the present embodiment, a crosslinking agent having at least one allyl group may be used from the viewpoint of improvement in crosslinkability. Specific examples of the crosslinking agent having at least one allyl group include, but not limited to, allylphenols such as 2,2-bis(3-allyl-4-hydroxyphenyl)propane, 1,1,1,3,3,3-hexafluoro-2,2-bis(3-allyl-4-hydroxyphenyl)propane, bis(3-allyl-4-hydroxyphenyl)sulfone, bis(3-allyl-4-hydroxyphenyl) sulfide, and bis(3-allyl-4-hydroxyphenyl) ether, allyl cyanates such as 2,2-bis(3-allyl-4-cyanatophenyl)propane, 1,1,1,3,3,3-hexafluoro-2,2-bis(3-allyl-4-cyanatophenyl)propane, bis(3-allyl-4-cyanatophenyl)sulfone, bis(3-allyl-4-cyanatophenyl) sulfide, and bis(3-allyl-4-cyanatophenyl) ether, diallyl phthalate, diallyl isophthalate, diallyl terephthalate, triallyl isocyanurate, trimethylolpropane diallyl ether, and pentaerythritol allyl ether. These crosslinking agents may be alone, or may be a mixture of two or more kinds. Among them, an allylphenol such as 2,2-bis(3-allyl-4-hydroxyphenyl)propane, 1,1,1,3,3,3-hexafluoro-2,2-bis(3-allyl-4-hydroxyphenyl)propane, bis(3-allyl-4-hydroxyphenyl)sulfone, bis(3-allyl-4-hydroxyphenyl) sulfide and bis(3-allyl-4-hydroxyphenyl) ether is preferable from the viewpoint of excellent compatibility with a bismaleimide compound and/or an addition polymerization-type maleimide resin.

With the film forming material for lithography of the present embodiment, the film for lithography of the present embodiment can be formed by crosslinking and curing the bismaleimide compound and/or the addition polymerization-type maleimide resin alone, or after compounding with the above crosslinking agent. Examples of the crosslinking method include approaches such as heat curing and photocuring.

The content ratio of the crosslinking agent is normally in the range of 0.1 to 10000 parts by mass based on 100 parts by mass of the total mass of the bismaleimide compound and the addition polymerization-type maleimide resin, preferably in the range of 0.1 to 1000 parts by mass from the viewpoint of heat resistance and solubility, more preferably in the range of 0.1 to 100 parts by mass, further preferably in the range of 1 to 50 parts by mass, and most preferably in the range of 1 to 30 parts by mass.

In the film forming material for lithography of the present embodiment, if required, a crosslinking promoting agent for accelerating crosslinking and curing reaction can be used.

The crosslinking promoting agent is not particularly limited as long as the crosslinking promoting agent accelerates crosslinking or curing reaction, and examples thereof include amines, imidazoles, organic phosphines, and Lewis acids. These crosslinking promoting agents can be used alone as one kind or can be used in combination of two or more kinds. Among them, an imidazole or an organic phosphine is preferable, and an imidazole is more preferable from the viewpoint of decrease in crosslinking temperature.

Examples of the crosslinking promoting agent include, but are not limited to, tertiary amines such as 1,8-diazabicyclo(5,4,0)undecene-7, triethylenediamine, benzyldimethylamine, triethanolamine, dimethylaminoethanol, and tris(dimethylaminomethyl)phenol; imidazoles such as 2-methylimidazole, 2-phenylimidazole, 2-ethyl-4-methylimidazole, 2-phenyl-4-methylimidazole, 2-heptadecylimidazole, and 2,4,5-triphenylimidazole; organic phosphines such as tributylphosphine, methyldiphenylphosphine, triphenylphosphine, diphenylphosphine, and phenylphosphine; tetra substituted phosphonium-tetra substituted borates such as tetraphenylphosphonium-tetraphenyl borate, tetraphenylphosphonium-ethyltriphenyl borate, and tetrabutylphosphonium-tetrabutyl borate; and tetraphenylboron salts such as 2-ethyl-4-methylimidazole-tetraphenyl borate and N-methylmorpholine-tetraphenyl borate.

The amount of the crosslinking promoting agent to be compounded is usually preferably in the range of 0.1 to 10 parts by mass based on 100 parts by mass of the total mass of the bismaleimide compound and the addition polymerization-type maleimide resin, and is more preferably in the range of 0.1 to 5 parts by mass and still more preferably in the range of 0.1 to 3 parts by mass, from the viewpoint of easy control and cost efficiency.

### <Radical polymerization initiator>

The film forming material for lithography of the present embodiment can contain, if required, a radical polymerization initiator. The radical polymerization initiator may be a photopolymerization initiator that initiates radical polymerization by light, or may be a thermal polymerization initiator that initiates radical polymerization by heat.

Such a radical polymerization initiator is not particularly limited, and a radical polymerization initiator conventionally used can be arbitrarily adopted. Examples thereof include ketone-based photopolymerization initiators such as 1-hydroxy cyclohexyl phenyl ketone, benzyl dimethyl ketal, 2-hydroxy-2-methyl-1-phenylpropan-1-one, 1-[4-(2-hydroxyethoxy)-phenyl]-2-hydroxy-2-methyl-1-propan-1-one, 2-hydroxy-1-{4-[4-(2-hydroxy-2-methylpropionyl)-benzyl]phenyl}-2-methylpropan-1-one, 2,4,6-trimethylbenzoyl-diphenyl-phosphine oxide, and bis(2,4,6-trimethylbenzoyl)-phenylphosphine oxide; and organic peroxide-based polymerization initiators such as methyl ethyl ketone peroxide, cyclohexanone peroxide, methylcyclohexanone peroxide, methyl acetoacetate peroxide, acetyl acetate peroxide, 1,1-bis(t-hexylperoxy)-3,3,5-trimethylcyclohexane, 1,1-bis(t-hexylperoxy)-cyclohexane, 1,1-bis(t-butylperoxy)-3,3,5-trimethylcyclohexane, 1,1-bis(t-butylperoxy)-2-methylcyclohexane, 1,1-bis(t-butylperoxy)-cyclohexane, 1,1-bis(t-butylperoxy)cyclododecane, 1,1-bis(t-butylperoxy)butane, 2,2-bis(4,4-di-t-butylperoxycyclohexyl)propane, p-menthane hydroperoxide, diisopropylbenzene hydroperoxide, 1,1,3,3-tetramethylbutyl hydroperoxide, cumene hydroperoxide, t-hexyl hydroperoxide, t-butyl hydroperoxide, α,α'-bis(t-butylperoxy)diisopropylbenzene, dicumyl peroxide, 2,5-dimethyl-2,5-bis(t-butylperoxy)hexane, t-butylcumyl peroxide, di-t-butyl peroxide, 2,5-dimethyl-2,5-bis(t-butylperoxy)hexyne-3, isobutyryl peroxide, 3,5,5-trimethylhexanoyl peroxide, octanoyl peroxide, lauroyl peroxide, stearoyl peroxide, succinic acid peroxide, m-toluoyl benzoyl peroxide, benzoyl peroxide, di-n-propyl peroxydicarbonate, diisopropyl peroxydicarbonate, bis(4-t-butylcyclohexyl) peroxydicarbonate, di-2-ethoxyethyl peroxydicarbonate, di-2-ethoxyhexyl peroxydicarbonate, di-3-methoxybutyl peroxydicarbonate, di-s-butyl peroxydicarbonate, di(3-methyl-3-methoxybutyl) peroxydicarbonate, α,α'-bis(neodecanoylperoxy)diisopropylbenzene, cumyl peroxyneodecanoate, 1,1,3,3-tetramethylbutyl peroxyneodecanoate, 1-cyclohexyl-1-methylethyl peroxyneodecanoate, t-hexyl peroxyneodecanoate, t-butyl peroxyneodecanoate, t-hexyl peroxypivalate, t-butyl peroxypivalate, 1,1,3,3-tetramethylbutyl peroxy-2-ethylhexanoate, 2,5-dimethyl-2,5-bis(2-ethylhexanoylperoxy)hexanoate, 1-cyclohexyl-1-methylethyl peroxy-2-ethylhexanoate, t-hexyl peroxy-2-ethylhexanoate, t-butyl peroxy-2-ethylhexanoate, t-hexyl peroxyisopropylmonocarbonate, t-butyl peroxyisobutyrate, t-butyl peroxymalate, t-butyl peroxy-3,5,5-trimethylhexanoate, t-butyl peroxylaurate, t-butyl peroxyisopropylmonocarbonate, t-butyl peroxy-2-ethylhexylmonocarbonate, t-butyl peroxyacetate, t-butyl peroxy-m-toluylbenzoate, t-butyl peroxybenzoate, bis(t-butylperoxy) isophthalate, 2,5-dimethyl-2,5-bis(m-toluylperoxy)hexane, t-hexyl peroxybenzoate, 2,5-dimethyl-2,5-bis(benzoylperoxy)hexane, t-butyl peroxyallylmonocarbonate, t-butyltrimethylsilyl peroxide, 3,3',4,4'-tetra(t-butylperoxycarbonyl)benzophenone, and 2,3-dimethyl-2,3-diphenylbutane.

Moreover, examples thereof include azo-based polymerization initiators such as 2-phenylazo-4-methoxy-2,4-dimethylvaleronitrile, 1-[(1-cyano-1-methylethyl)azo]formamide, 1,1'-azobis(cyclohexane-1-carbonitrile), 2,2'-azobis(2-methylbutyronitrile), 2,2'-azobisisobutyronitrile, 2,2'-azobis(2,4-dimethylvaleronitrile), 2,2'-azobis(2-methylpropionamidine) dihydrochloride, 2,2'-azobis(2-methyl-N-phenylpropionamidine) dihydrochloride, 2,2'-azobis[N-(4-chlorophenyl)-2-methylpropionamidine]dihydride chloride, 2,2'-azobis[N-(4-hydrophenyl)-2-methylpropionamidine] dihydrochloride, 2,2'-azobis[2-methyl-N-(phenylmethyl)propionamidine] dihydrochloride, 2,2'-azobis[2-methyl-N-(2-propenyl)propionamidine] dihydrochloride, 2,2'-azobis[N-(2-hydroxyethyl)-2-methylpropionamidine] dihydrochloride, 2,2'-azobis[2-(5-methyl-2-imidazolin-2-yl)propane] dihydrochloride, 2,2'-azobis[2-(2-imidazolin-2-yl)propane] dihydrochloride, 2,2'-azobis[2-(4,5,6,7-tetrahydro-1H-1,3-diazepin-2-yl)propane] dihydrochloride, 2,2'-azobis[2-(3,4,5,6-tetrahydropyrimidin-2-yl)propane] dihydrochloride, 2,2'-azobis[2-(5-hydroxy-3,4,5,6-tetrahydropyrimidin-2-yl)propane] dihydrochloride, 2,2'-azobis[2-[1-(2-hydroxyethyl)-2-imidazolin-2-yl]propane] dihydrochloride, 2,2'-azobis[2-(2-imidazolin-2-yl)propane], 2,2'-azobis[2-methyl-N-[1,1-bis(hydroxymethyl)-2-hydroxyethyl]propionamide], 2,2'-azobis[2-methyl-N-[1,1-bis(hydroxymethyl)ethyl]propionamide], 2,2'-azobis[2-methyl-N-(2-hydroxyethyl)propionamide], 2,2'-azobis(2-methylpropionamide), 2,2'-azobis(2,4,4-trimethylpentane), 2,2'-azobis(2-methylpropane), dimethyl-2,2-azobis(2-methylpropionate), 4,4'-azobis(4-cyanopentanoic acid), and 2,2'-azobis[2-(hydroxymethyl)propionitrile]. As the radical polymerization initiator of the present embodiment, one kind thereof may be used alone, or two or more kinds may be used in combination. Alternatively, the radical polymerization initiator of the present embodiment may be used in further combination with an additional known polymerization initiator.

The content of the above radical polymerization initiator may be any amount as long as it is a stoichiometrically required amount relative to the total mass of the bismaleimide compound and the addition polymerization-type maleimide resin, but it is preferably 0.05 to 25 parts by mass and more preferably 0.1 to 10 parts by mass, based on 100 parts by mass of the total mass of the bismaleimide compound and the addition polymerization-type maleimide resin. When the content of the radical polymerization initiator is 0.05 part by mass or more, there is a tendency that curing of the bismaleimide compound and/or the maleimide resin can be prevented from being insufficient. On the other hand, when the content of the radical polymerization initiator is 25 parts by mass or less, there is a tendency that the long term storage stability of the film forming material for lithography at room temperature can be prevented from being impaired.

### [Method for purifying film forming material for lithography]

The film forming material for lithography can be purified by washing with an acidic aqueous solution. The above purification method comprises a step in which the film forming material for lithography is dissolved in an organic solvent that is incompatible with water to obtain an organic phase, the organic phase is brought into contact with an acidic aqueous solution to carry out extraction treatment (a first extraction step), thereby transferring metals contained in the organic phase containing the film forming material for lithography and the organic solvent to an aqueous phase, and then, the organic phase and the aqueous phase are separated. According to the purification, the contents of various metals in the film forming material for lithography of the present invention can be reduced remarkably.

The organic solvent that is incompatible mix with water is not particularly limited, but is preferably an organic solvent that is safely applicable to semiconductor manufacturing processes. Normally, the amount of the organic solvent used is approximately 1 to 100 times by mass relative to the compound used.

Specific examples of the organic solvent to be used include those described in International Publication No. WO 2015/080240. Among these, toluene, 2-heptanone, cyclohexanone, cyclopentanone, methyl isobutyl ketone, propylene glycol monomethyl ether acetate, ethyl acetate, and the like are preferable, and cyclohexanone and propylene glycol monomethyl ether acetate are particularly preferable. These organic solvents can be each used alone, and can be used as a mixture of two or more kinds.

The above acidic aqueous solution is appropriately selected from aqueous solutions in which generally known organic or inorganic compounds are dissolved in water. For example, examples thereof include those described in International Publication No. WO 2015/080240. These acidic aqueous solutions can be each used alone, and can be also used as a combination of two or more kinds. Examples of the acidic aqueous solution may include, for example, an aqueous mineral acid solution and an aqueous organic acid solution. Examples of the aqueous mineral acid solution may include, for example, an aqueous solution comprising one or more selected from the group consisting of hydrochloric acid, sulfuric acid, nitric acid and phosphoric acid. Examples of the aqueous organic acid solution may include, for example, an aqueous solution comprising one or more selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid and trifluoroacetic acid. Moreover, as the acidic aqueous solution, aqueous solutions of sulfuric acid, nitric acid, and a carboxylic acid such as acetic acid, oxalic acid, tartaric acid and citric acid are preferable, aqueous solutions of sulfuric acid, oxalic acid, tartaric acid and citric acid are further preferable, and an aqueous solution of oxalic acid is particularly preferable. It is considered that polyvalent carboxylic acids such as oxalic acid, tartaric acid and citric acid coordinate with metal ions and provide a chelating effect, and thus are capable of removing more metals. Water used herein is preferably water, the metal content of which is small, such as ion exchanged water, according to the purpose of the present invention.

The pH of the acidic aqueous solution is not particularly limited, but when the acidity of the aqueous solution is too high, it may have a negative influence on the used compound or resin, which is not preferable. Normally, the pH range is about 0 to 5, and is more preferably about pH 0 to 3.

The amount of the acidic aqueous solution used is not particularly limited, but when the amount is too small, it is required to increase the number of extraction treatments for removing metals, and on the other hand, when the amount of the aqueous solution is too large, the entire fluid volume becomes large, which may cause operational problems. Normally, the amount of the aqueous solution used is 10 to 200 parts by mass and preferably 20 to 100 parts by mass relative to the solution of the film forming material for lithography.

By bringing the acidic aqueous solution into contact with a solution (B) containing the film forming material for lithography and the organic solvent that is incompatible with water, metals can be extracted.

The temperature at which the above extraction treatment is carried out is generally in the range of 20 to 90°C, and preferably 30 to 80°C. The extraction operation is carried out, for example, by thoroughly mixing the solution (B) and the acidic aqueous solution by stirring or the like and then leaving the obtained mixed solution to stand still. Thereby, metals contained in the solution containing the used compound and the organic solvent are transferred to the aqueous phase. Also, by this operation, the acidity of the solution is lowered, and the deterioration of the used compound can be suppressed.

After the extraction treatment, the mixed solution is separated into a solution phase containing the used compound and the organic solvent and an aqueous phase, and the solution containing the organic solvent is recovered by decantation or the like. The time for leaving the mixed solution to stand still is not particularly limited, but when the time for leaving the mixed solution to stand still is too short, separation of the solution phase containing the organic solvent and the aqueous phase becomes poor, which is not preferable. Normally, the time for leaving the mixed solution to stand still is 1 minute or longer, more preferably 10 minutes or longer, and further preferably 30 minutes or longer. While the extraction treatment may be carried out once, it is effective to repeat mixing, leaving-to-stand-still, and separating operations multiple times.

When such an extraction treatment is carried out using the acidic aqueous solution, after the treatment, it is preferable to further subjecting the recovered organic phase that has been extracted from the aqueous solution and contains the organic solvent to an extraction treatment with water (a second extraction step). The extraction operation is carried out by thoroughly mixing the organic phase and water by stirring or the like and then leaving the obtained mixed solution to stand still. The resultant mixed solution is separated into a solution phase containing the compound and the organic solvent and an aqueous phase, and thus the solution phase is recovered by decantation or the like. Water used herein is preferably water, the metal content of which is small, such as ion exchanged water, according to the purpose of the present invention. While the extraction treatment may be carried out once, it is effective to repeat mixing, leaving-to-stand-still, and separating operations multiple times. The proportions of both used in the extraction treatment and temperature, time, and other conditions are not particularly limited, and may be the same as those of the previous contact treatment with the acidic aqueous solution.

Water that is unwantedly present in the thus-obtained solution containing the film forming material for lithography and the organic solvent can be easily removed by performing vacuum distillation or a like operation. Also, if required, the concentration of the compound can be regulated to be any concentration by adding an organic solvent.

A method for only obtaining the film forming material for lithography from the obtained solution containing the organic solvent can be carried out through a known method such as reduced-pressure removal, separation by reprecipitation, and a combination thereof. Known treatments such as concentration operation, filtration operation, centrifugation operation, and drying operation can be carried out if required.

### [Composition for film formation for lithography]

A composition for film formation for lithography of the present embodiment comprises the above film forming material for lithography and a solvent. The film for lithography is, for example, an underlayer film for lithography.

The composition for film formation for lithography of the present embodiment can form a desired cured film by applying it on a base material, subsequently heating it to evaporate the solvent if necessary, and then heating or photoirradiating it. A method for applying the composition for film formation for lithography of the present embodiment is arbitrary, and a method such as spin coating, dipping, flow coating, inkjet coating, spraying, bar coating, gravure coating, slit coating, roll coating, transfer printing, brush coating, blade coating and air knife coating can be employed appropriately.

The temperature at which the film is heated is not particularly limited according to the purpose of evaporating the solvent, and the heating can be carried out at, for example, 40 to 400°C. A method for heating is not particularly limited, and for example, the solvent may be evaporated under an appropriate atmosphere such as atmospheric air, an inert gas including nitrogen and vacuum by using a hot plate or an oven. For the heating temperature and heating time, it is only required to select conditions suitable for a processing step for an electronic device that is aimed at and to select heating conditions by which physical property values of the obtained film satisfy requirements of the electronic device. Conditions for photoirradiation are not particularly limited, either, and it is only required to employ appropriate irradiation energy and irradiation time depending on a film forming material for lithography to be used.

### <Solvent>

A solvent to be used in the composition for film formation for lithography of the present embodiment is not particularly limited as long as it can at least dissolve bismaleimide and/or an addition polymerization-type maleimide resin, and any known solvent can be used appropriately.

Specific examples of the solvent include those described in International Publication No. WO 2013/024779. These solvents can be used alone as one kind or used in combination of two or more kinds.

Among the above solvents, cyclohexanone, propylene glycol monomethyl ether, propylene glycol monomethyl ether acetate, ethyl lactate, methyl hydroxyisobutyrate or anisole is particularly preferable from the viewpoint of safety.

The content of the solvent is not particularly limited and is preferably 25 to 9,900 parts by mass, more preferably 400 to 7,900 parts by mass, and further preferably 900 to 4,900 parts by mass based on 100 parts by mass of the total mass of the bismaleimide compound and the addition polymerization-type maleimide resin in the material for film formation for lithography, from the viewpoint of solubility and film formation.

### <Acid Generating Agent>

The composition for film formation for lithography of the present embodiment may contain an acid generating agent, if required, from the viewpoint of, for example, further accelerating crosslinking reaction. An acid generating agent that generates an acid by thermal decomposition, an acid generating agent that generates an acid by light irradiation, and the like are known, any of which can be used.

Examples of the acid generating agent include, for example, those described in International Publication No. WO 2013/024779. Among them, in particular, onium salts such as di-tertiary-butyl diphenyliodonium nonafluoromethanesulfonate, diphenyliodonium trifluoromethanesulfonate, (p-tert-butoxyphenyl)phenyliodonium trifluoromethanesulfonate, diphenyliodonium p-toluenesulfonate, (p-tert-butoxyphenyl)phenyliodonium p-toluenesulfonate, triphenylsulfonium trifluoromethanesulfonate, (p-tert-butoxyphenyl)diphenylsulfonium trifluoromethanesulfonate, tris(p-tert-butoxyphenyl) sulfonium trifluoromethanesulfonate, triphenylsulfonium p-toluenesulfonate, (p-tert-butoxyphenyl)diphenylsulfonium p-toluenesulfonate, tris(p-tert-butoxyphenyl)sulfonium p-toluenesulfonate, trinaphthylsulfonium trifluoromethanesulfonate, cyclohexylmethyl(2-oxocyclohexyl)sulfonium trifluoromethanesulfonate, (2-norbornyl)methyl(2-oxocyclohexyl) sulfonium trifluoromethanesulfonate and 1,2'-naphthylcarbonylmethyltetrahydrothiophenium triflate; diazomethane derivatives such as bis(benzenesulfonyl)diazomethane, bis(p-toluenesulfonyl)diazomethane, bis(cyclohexylsulfonyl)diazomethane, bis(n-butylsulfonyl)diazomethane, bis(isobutylsulfonyl)diazomethane, bis(sec-butylsulfonyl)diazomethane, bis(n-propylsulfonyl)diazomethane, bis(isopropylsulfonyl)diazomethane and bis(tert-butylsulfonyl)diazomethane; glyoxime derivatives such as bis-(p-toluenesulfonyl)-α-dimethylglyoxime and bis-(n-butanesulfonyl)-α-dimethylglyoxime; bissulfone derivatives such as bisnaphthylsulfonylmethane; sulfonic acid ester derivatives of N-hydroxyimide compounds such as N-hydroxysuccinimide methanesulfonic acid ester, N-hydroxysuccinimide trifluoromethanesulfonic acid ester, N-hydroxysuccinimide 1-propanesulfonic acid ester, N-hydroxysuccinimide 2-propanesulfonic acid ester, N-hydroxysuccinimide 1-pentanesulfonic acid ester, N-hydroxysuccinimide p-toluenesulfonic acid ester, N-hydroxynaphthalimido methanesulfonic acid ester and N-hydroxynaphthalimido benzenesulfonic acid ester are preferably used.

The content of the acid generating agent in the composition for film formation for lithography of the present embodiment is not particularly limited, and is preferably 0 to 50 parts by mass and more preferably 0 to 40 parts by mass based on 100 parts by mass of total mass of the bismaleimide compound and the addition polymerization-type maleimide resin in the film forming material for lithography. By the preferable range mentioned above, crosslinking reaction tends to be enhanced. Also, a mixing event with a resist layer tends to be prevented.

### [Basic compound]

The composition for underlayer film formation for lithography of the present embodiment may further contain a basic compound from the viewpoint of, for example, improving storage stability.

The above basic compound plays a role as a quencher against acids in order to prevent crosslinking reaction from proceeding due to a trace amount of an acid generated by the acid generating agent. Examples of such a basic compound include, but are not limited to, for example, primary, secondary or tertiary aliphatic amines, amine blends, aromatic amines, heterocyclic amines, nitrogen-containing compounds having a carboxy group, nitrogen-containing compounds having a sulfonyl group, nitrogen-containing compounds having a hydroxy group, nitrogen-containing compounds having a hydroxyphenyl group, alcoholic nitrogen-containing compounds, amide derivatives or imide derivatives, described in International Publication No. WO 2013-024779.

The content of the basic compound in the composition for film formation for lithography of the present embodiment is not particularly limited and is preferably 0 to 2 parts by mass and more preferably 0 to 1 part by mass based on 100 parts by mass of the total mass of the bismaleimide compound and the addition polymerization-type maleimide resin in the film forming material for lithography. By the preferable range mentioned above, storage stability tends to be enhanced without excessively deteriorating crosslinking reaction.

The composition for film formation for lithography of the present embodiment may further contain a known additive agent. Examples of the known additive agent include, but are not limited to, ultraviolet absorbers, antifoaming agents, colorants, pigments, nonionic surfactants, anionic surfactants and cationic surfactants.

### [Method for forming underlayer film for lithography and pattern]

The underlayer film for lithography of the present embodiment is formed by using the composition for film formation for lithography of the present embodiment.

A pattern formation method of the present embodiment has the steps of: forming an underlayer film on a supporting material using the composition for film formation for lithography of the present embodiment (step (A-1)); forming at least one photoresist layer on the underlayer film (step (A-2)); and after the step (A-2), irradiating a predetermined region of the photoresist layer with radiation for development (step (A-3)).

Furthermore, another pattern formation method of the present embodiment has the steps of: forming an underlayer film on a supporting material using the composition for film formation for lithography of the present embodiment (step (B-1)); forming an intermediate layer film on the underlayer film using a resist intermediate layer film material containing a silicon atom (step (B-2)); forming at least one photoresist layer on the intermediate layer film (step (B-3)); after the step (B-3), irradiating a predetermined region of the photoresist layer with radiation for development, thereby forming a resist pattern (step (B-4)); and after the step (B-4), etching the intermediate layer film with the resist pattern as a mask, etching the underlayer film with the obtained intermediate layer film pattern as an etching mask, and etching the supporting material with the obtained underlayer film pattern as an etching mask, thereby forming a pattern on the supporting material (step (B-5)).

The underlayer film for lithography of the present embodiment is not particularly limited by its formation method as long as it is formed from the composition for film formation for lithography of the present embodiment. A known approach can be applied thereto. The underlayer film can be formed by, for example, applying the composition for film formation for lithography of the present embodiment onto a supporting material by a known coating method or printing method such as spin coating or screen printing, and then removing an organic solvent by volatilization or the like.

It is preferable to perform baking in the formation of the underlayer film, for preventing a mixing event with an upper layer resist while accelerating crosslinking reaction. In this case, the baking temperature is not particularly limited and is preferably in the range of 80 to 450°C, and more preferably 200 to 400°C. The baking time is not particularly limited and is preferably in the range of 10 to 300 seconds. The thickness of the underlayer film can be arbitrarily selected according to required performances and is not particularly limited, but is preferably 30 to 20,000 nm, more preferably 50 to 15,000 nm, and further preferably 50 to 1000 nm.

After preparing the underlayer film on the supporting material, in the case of a two-layer process, it is preferable to prepare a silicon-containing resist layer or a usual single-layer resist composed of hydrocarbon thereon, and in the case of a three-layer process, it is preferable to prepare a silicon-containing intermediate layer thereon and further prepare a single-layer resist layer not containing silicon thereon. In this case, for a photoresist material for forming this resist layer, a known material can be used.

For the silicon-containing resist material for a two-layer process, a silicon atom-containing polymer such as a polysilsesquioxane derivative or a vinylsilane derivative is used as a base polymer, and a positive type photoresist material further containing an organic solvent, an acid generating agent, and if required, a basic compound or the like is preferably used, from the viewpoint of oxygen gas etching resistance. Herein, a known polymer that is used in this kind of resist material can be used as the silicon atom-containing polymer.

A polysilsesquioxane-based intermediate layer is preferably used as the silicon-containing intermediate layer for a three-layer process. By imparting effects as an antireflection film to the intermediate layer, there is a tendency that reflection can be effectively suppressed. For example, use of a material containing a large amount of an aromatic group and having high supporting material etching resistance as the underlayer film in a process for exposure at 193 nm tends to increase a k value and enhance supporting material reflection. However, the intermediate layer suppresses the reflection so that the supporting material reflection can be 0.5% or less. The intermediate layer having such an antireflection effect is not limited, and polysilsesquioxane that crosslinks by an acid or heat in which a light absorbing group having a phenyl group or a silicon-silicon bond is introduced is preferably used for exposure at 193 nm.

Alternatively, an intermediate layer formed by chemical vapour deposition (CVD) may be used. The intermediate layer highly effective as an antireflection film prepared by CVD is not limited, and, for example, a SiON film is known. In general, the formation of an intermediate layer by a wet process such as spin coating or screen printing is more convenient and more advantageous in cost, as compared with CVD. The upper layer resist for a three-layer process may be positive type or negative type, and the same as a single-layer resist generally used can be used.

The underlayer film of the present embodiment can also be used as an antireflection film for usual single-layer resists or an underlying material for suppression of pattern collapse. The underlayer film of the present embodiment is excellent in etching resistance for an underlying process and can be expected to also function as a hard mask for an underlying process.

In the case of forming a resist layer from the above photoresist material, a wet process such as spin coating or screen printing is preferably used, as in the case of forming the above underlayer film. After coating with the resist material by spin coating or the like, prebaking is generally performed. This prebaking is preferably performed at 80 to 180°C in the range of 10 to 300 seconds. Then, exposure, post-exposure baking (PEB), and development can be performed according to a conventional method to obtain a resist pattern. The thickness of the resist film is not particularly limited, and in general, is preferably 30 to 500 nm and more preferably 50 to 400 nm.

The exposure light can be arbitrarily selected and used according to the photoresist material to be used. General examples thereof can include a high energy ray having a wavelength of 300 nm or less, specifically, excimer laser of 248 nm, 193 nm, or 157 nm, soft x-ray of 3 to 20 nm, electron beam, and X-ray.

In a resist pattern formed by the method mentioned above, pattern collapse is suppressed by the underlayer film of the present embodiment. Therefore, use of the underlayer film of the present embodiment can produce a finer pattern and can reduce an exposure amount necessary for obtaining the resist pattern.

Next, etching is performed with the obtained resist pattern as a mask. Gas etching is preferably used as the etching of the underlayer film in a two-layer process. The gas etching is preferably etching using oxygen gas. In addition to oxygen gas, an inert gas such as He or Ar, or CO, CO₂, NH₃, SO₂, N₂, NO₂, or H₂ gas may be added. Alternatively, the gas etching may be performed with CO, CO₂, NH₃, N₂, NO₂, or H₂ gas without the use of oxygen gas. Particularly, the latter gas is preferably used for side wall protection in order to prevent the undercut of pattern side walls.

On the other hand, gas etching is also preferably used as the etching of the intermediate layer in a three-layer process. The same gas etching as described in the two-layer process mentioned above is applicable. Particularly, it is preferable to process the intermediate layer in a three-layer process by using chlorofluorocarbon-based gas and using the resist pattern as a mask. Then, as mentioned above, the underlayer film can be processed by, for example, oxygen gas etching with the intermediate layer pattern as a mask.

Herein, in the case of forming an inorganic hard mask intermediate layer film as the intermediate layer, a silicon oxide film, a silicon nitride film, or a silicon oxynitride film (SiON film) is formed by CVD, ALD, or the like. A method for forming the nitride film is not limited, and for example, a method described in Japanese Patent Laid-Open No. 2002-334869 (Patent Literature 6) or WO 2004/066377 (Patent Literature 7) can be used. Although a photoresist film can be formed directly on such an intermediate layer film, an organic antireflection film (BARC) may be formed on the intermediate layer film by spin coating and a photoresist film may be formed thereon.

A polysilsesquioxane-based intermediate layer is preferably used as the intermediate layer. By imparting effects as an antireflection film to the resist intermediate layer film, there is a tendency that reflection can be effectively suppressed. A specific material for the polysilsesquioxane-based intermediate layer is not limited, and, for example, a material described in Japanese Patent Laid-Open No. 2007-226170 (Patent Literature 8) or Japanese Patent Laid-Open No. 2007-226204 (Patent Literature 9) can be used.

The subsequent etching of the supporting material can also be performed by a conventional method. For example, the supporting material is made of SiO₂ or SiN can be etched mainly using chlorofluorocarbon-based gas, and the supporting material made of p-Si, Al or W can be etched mainly using chlorine- or bromine-based gas. In the case of etching the supporting material with chlorofluorocarbon-based gas, the silicon-containing resist of the two-layer resist process or the silicon-containing intermediate layer of the three-layer process is peeled at the same time with supporting material processing. On the other hand, in the case of etching the supporting material with chlorine- or bromine-based gas, the silicon-containing resist layer or the silicon-containing intermediate layer is separately peeled and in general, peeled by dry etching using chlorofluorocarbon-based gas after supporting material processing.

A feature of the underlayer film of the present embodiment is that it is excellent in etching resistance of these supporting materials. The supporting material can be arbitrarily selected from known ones and used and is not particularly limited. Examples thereof include Si, α-Si, p-Si, SiO₂, SiN, SiON, W, TiN, and Al. The supporting material may be a laminate having a film to be processed (supporting material to be processed) on a base material (support). Examples of such a film to be processed include various low-k films such as Si, SiO₂, SiON, SiN, p-Si, α-Si, W, W-Si, Al, Cu, and Al-Si, and stopper films thereof. A material different from that for the base material (support) is generally used. The thickness of the supporting material to be processed or the film to be processed is not particularly limited, and normally, it is preferably approximately 50 to 1,000,000 nm and more preferably 75 to 500,000 nm.

### Examples

Hereinafter, the present invention will be described in further detail with reference to Examples, Production Examples and Comparative Examples, but the present invention is not limited by these examples in any way.

### [Molecular weight]

The molecular weight of the synthesized compound was measured by LC-MS analysis using Acquity UPLC/MALDI-Synapt HDMS manufactured by Waters Corp.

### [Evaluation of heat resistance]

EXSTAR 6000 TG-DTA apparatus manufactured by SII NanoTechnology Inc. was used. About 5 mg of a sample was placed in an unsealed container made of aluminum, and the temperature was raised to 500°C at a temperature increase rate of 10°C/min in a nitrogen gas stream (100 ml/min), thereby measuring the amount of thermogravimetric weight loss. From a practical viewpoint, evaluation A or B described below is preferable. When the evaluation is A or B, the sample has high heat resistance and is applicable to high temperature baking.

### <Evaluation criteria>

A: The amount of thermogravimetric weight loss at 400°C is less than 10%
B: The amount of thermogravimetric weight loss at 400°C is 10% to 25%
C: The amount of thermogravimetric weight loss at 400°C is greater than 25%

### [Evaluation of solubility]

Propylene glycol monomethyl ether acetate (PGMEA) and the compound and/or the resin were added to a 50 ml screw bottle and stirred at 23°C for 1 hour using a magnetic stirrer. Then, the amount of the compound and/or the resin dissolved in PGMEA was measured and the result was evaluated according to the following criteria. From a practical viewpoint, evaluation A or B described below is preferable. When the evaluation is A or B, the sample has high storage stability in the solution state, and can be satisfyingly applied to an edge bead remover (mixed liquid of PGME/PGMEA) widely used for a fine processing process of semiconductors.

### <Evaluation criteria>

A: 10% by mass or more
B: 5% by mass or more and less than 10% by mass
C: less than 5% by mass

### (Synthetic Working Example 1) Synthesis of 4-APCH maleimide

A container (internal capacity: 100 ml) equipped with a stirrer, a condenser tube, and a burette was prepared. To this container, 2.66 g (10.0 mmol) of bis-(4-aminophenyl)cyclohexane (manufactured by Taoka Chemical Co., Ltd.), 2.15 g (22.0 mmol) of maleic anhydride (manufactured by KANTO CHEMICAL CO., INC.), 30 ml of dimethylformamide and 30 ml of m-xylene were added, and 0.4 g (2.3 mmol) of p-toluenesulfonic acid was added to prepare a reaction solution. The reaction solution was stirred at 120°C for 3.5 hours and reacted, and the produced water was recovered with a Dean-and-stark trap through azeotropic dehydration. Next, after cooling the reaction solution to 40°C, it was added dropwise into a beaker in which 300 ml of distilled water was placed to precipitate the product. After filtering the obtained slurry solution, the residue was washed with methanol and subjected to separation and purification with column chromatography to acquire 1.52 g of the target compound (4-APCH maleimide) represented by the following formula:

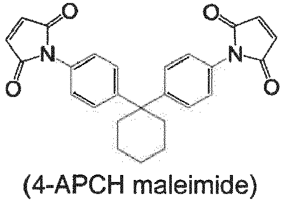

The following peaks were found by 400 MHz-¹H-NMR, and the compound was confirmed to have a chemical structure of the above formula.
¹H-NMR: (d-DMSO, internal standard TMS)
δ (ppm) 7.0-7.3 (8H, Ph-H), 3.9(4H, -CH=CH), 1.1-1.3(10H, -C6H10). As a result of measuring the molecular weight of the obtained compound by the above method, it was 426.

### (Synthetic Working Example 2) Synthesis of TPE-R maleimide

A container (internal capacity: 100 ml) equipped with a stirrer, a condenser tube, and a burette was prepared. To this container, 2.92 g (10.0 mmol) of 4,4'-(1,3-phenylenebis)oxydianiline (trade name: TPE-R, manufactured by Wakayama Seika Kogyo Co., Ltd.), 2.15 g (22.0 mmol) of maleic anhydride (manufactured by KANTO CHEMICAL CO., INC.), 30 ml of dimethylformamide and 30 ml of m-xylene were added, and 0.4 g (2.3 mmol) of p-toluenesulfonic acid was added to prepare a reaction solution. The reaction solution was stirred at 130°C for 4.0 hours to conduct reaction, and the produced water was recovered with a Dean-and-stark trap through azeotropic dehydration. Next, after cooling the reaction solution to 40°C, it was added dropwise into a beaker in which 300 ml of distilled water was placed to precipitate the product. After filtering the obtained slurry solution, the residue was washed with methanol and subjected to separation and purification with column chromatography to acquire 1.84 g of the target compound (TPE-R maleimide) represented by the following formula:

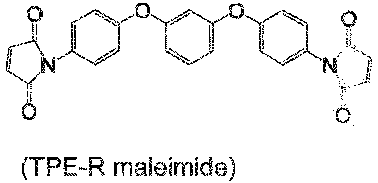

The following peaks were found by 400 MHz-¹H-NMR, and the compound was confirmed to have a chemical structure of the above formula.
¹H-NMR: (d-DMSO, internal standard TMS)
δ (ppm) 6.8-7.5 (12H, Ph-H), 3.4 (4H, -CH=CH). As a result of measuring the molecular weight of the obtained compound by the above method, it was 452.

### (Synthetic Working Example 3) Synthesis of HFBAPP maleimide

A container (internal capacity: 100 ml) equipped with a stirrer, a condenser tube, and a burette was prepared. To this container, 5.18 g (10.0 mmol) of (trade name: HFBAPP, manufactured by Wakayama Seika Kogyo Co., Ltd.), 2.15 g (22.0 mmol) of maleic anhydride (manufactured by KANTO CHEMICAL CO., INC.), 30 ml of dimethylformamide and 30 ml of m-xylene were added, and 0.4 g (2.3 mmol) of p-toluenesulfonic acid was added to prepare a reaction solution. The reaction solution was stirred at 130°C for 5.0 hours and reacted, and the produced water was recovered with a Dean-and-stark trap through azeotropic dehydration. Next, after cooling the reaction solution to 40°C, it was added dropwise into a beaker in which 300 ml of distilled water was placed to precipitate the product. After filtering the obtained slurry solution, the residue was washed with methanol and subjected to separation and purification with column chromatography to acquire 3.5 g of the target compound (HFBAPP maleimide) represented by the following formula:

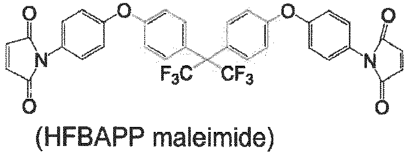

The following peaks were found by 400 MHz-¹H-NMR, and the compound was confirmed to have a chemical structure of the above formula.
¹H-NMR: (d-DMSO, internal standard TMS)
δ (ppm) 6.6-7.4 (16H, Ph-H), 3.4 (4H, -CH=CH)

As a result of measuring the molecular weight of the obtained compound by the above method, it was 678.

### (Synthetic Working Example 4) Synthesis of TFMB maleimide

A container (internal capacity: 100 ml) equipped with a stirrer, a condenser tube, and a burette was prepared. To this container, 3.21 g (10.0 mmol) of (trade name: TFMB, manufactured by Wakayama Seika Kogyo Co., Ltd.), 2.15 g (22.0 mmol) of maleic anhydride (manufactured by KANTO CHEMICAL CO., INC.), 40 ml of dimethylformamide and 30 ml of m-xylene were added, and 0.4 g (2.3 mmol) of p-toluenesulfonic acid was added to prepare a reaction solution. The reaction solution was stirred at 130°C for 4.0 hours to conduct reaction, and the produced water was recovered with a Dean-and-stark trap through azeotropic dehydration. Next, after cooling the reaction solution to 40°C, it was added dropwise into a beaker in which 300 ml of distilled water was placed to precipitate the product. After filtering the obtained slurry solution, the residue was washed with methanol and subjected to separation and purification with column chromatography to acquire 2.4 g of the target compound (TFMB maleimide) represented by the following formula:

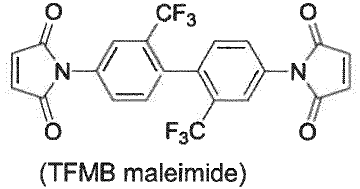

The following peaks were found by 400 MHz-¹H-NMR, and the compound was confirmed to have a chemical structure of the above formula.
¹H-NMR: (d-DMSO, internal standard TMS)
δ (ppm) 6.6-7.1 (6H, Ph-H), 3.2 (4H, -CH=CH)

As a result of measuring the molecular weight of the obtained compound by the above method, it was 493.

### (Synthetic Working Example 5) Synthesis of DANPG maleimide

A container (internal capacity: 100 ml) equipped with a stirrer, a condenser tube, and a burette was prepared. To this container, 2.86 g (10.0 mmol) of (trade name: DANPG, manufactured by Wakayama Seika Kogyo Co., Ltd.), 2.15 g (22.0 mmol) of maleic anhydride (manufactured by KANTO CHEMICAL CO., INC.), 40 ml of dimethylformamide and 30 ml of m-xylene were added, and 0.4 g (2.3 mmol) of p-toluenesulfonic acid was added to prepare a reaction solution. The reaction solution was stirred at 130°C for 4.0 hours to conduct reaction, and the produced water was recovered with a Dean-and-stark trap through azeotropic dehydration. Next, after cooling the reaction solution to 40°C, it was added dropwise into a beaker in which 300 ml of distilled water was placed to precipitate the product. After filtering the obtained slurry solution, the residue was washed with methanol and subjected to separation and purification with column chromatography to acquire 2.7 g of the target compound (DANPG maleimide) represented by the following formula:

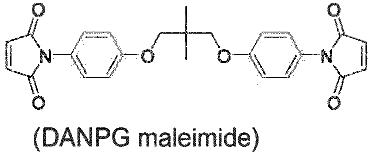

The following peaks were found by 400 MHz-¹H-NMR, and the compound was confirmed to have a chemical structure of the above formula.
¹H-NMR: (d-DMSO, internal standard TMS)
δ (ppm) 7.0-7.5 (8H, Ph-H), 3.2 (4H, -CH=CH) 2.4 (4H, -CH2-), 1.6-1.7 (6H, CH3-C-CH3)

As a result of measuring the molecular weight of the obtained compound by the above method, it was 446.

### <Example 1>

As a bismaleimide compound, 10 parts by mass of N,N'-biphenyl-based bismaleimide (BMI-70; manufactured by K·I Chemical Industry Co., LTD.) represented by the formula described below was used alone to prepare a film forming material for lithography.

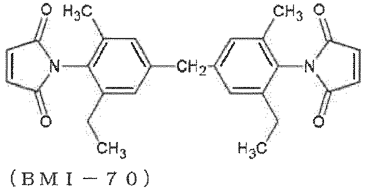

As a result of thermogravimetry, the amount of thermogravimetric weight loss at 400°C of the obtained film forming material for lithography was less than 10% (evaluation A). In addition, as a result of evaluation of solubility in PGMEA, the solubility was 5% by mass or more and less than 10% by mass (evaluation B), and the obtained film forming material for lithography was evaluated to have sufficient solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 2>

As an addition polymerization-type maleimide resin, 10 parts by mass of phenylmethane maleimide oligomer (BMI oligomer; BMI-2300; manufactured by Daiwa Kasei Industry Co., Ltd.) represented by the formula described below was used alone to prepare a film forming material for lithography.

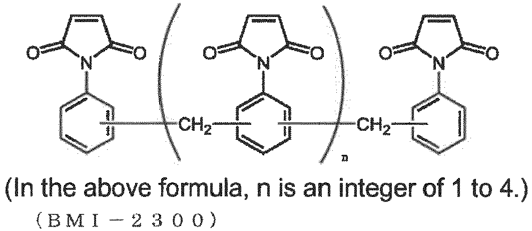

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 3>

As an addition polymerization maleimide-type resin, 10 parts by mass of a biphenyl aralkyl-based maleimide resin (MIR-3000-L, manufactured by Nippon Kayaku Co., Ltd.) represented by the formula described below was used alone to prepare a film forming material for lithography.

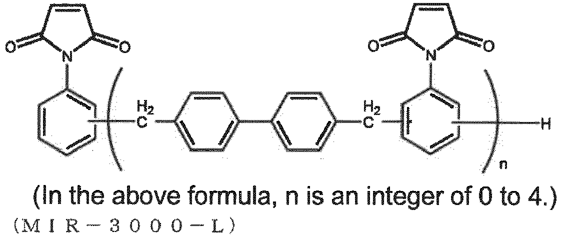

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 4>

As a bismaleimide compound, 10 parts by mass of N,N'-diphenylmethane-based bismaleimide (BMI-50P (n = 0 (dikaryon) proportion of 57.6%; manufactured by K·I Chemical Industry Co., LTD.)) including polynuclear matters represented by the formula described below was used alone to prepare a film forming material for lithography.

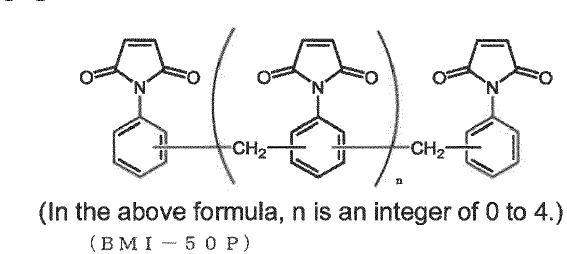

As a result of thermogravimetry, the amount of thermogravimetric weight loss at 400°C of the obtained film forming material for lithography was less than 10% (evaluation A). In addition, as a result of evaluation of solubility in PGMEA, the solubility was 5% by mass or more and less than 10% by mass (evaluation B), and the obtained film forming material for lithography was evaluated to have sufficient solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 5>

As a bismaleimide compound, 10 parts by mass of bismaleimide having an ester skeleton (BMI-1000P; manufactured by K·I Chemical Industry Co., LTD.) represented by the formula described below was used alone to prepare a film forming material for lithography.

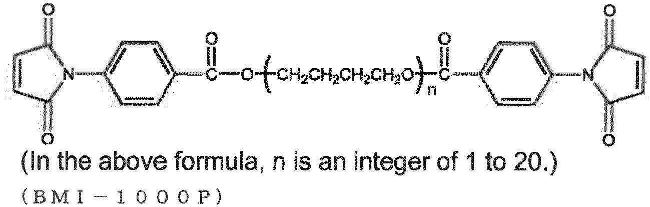

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have sufficient solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 6>

As a bismaleimide compound, 10 parts by mass of bismaleimide (BMI-80; manufactured by K·I Chemical Industry Co., LTD.) represented by the formula described below was used alone to prepare a film forming material for lithography.

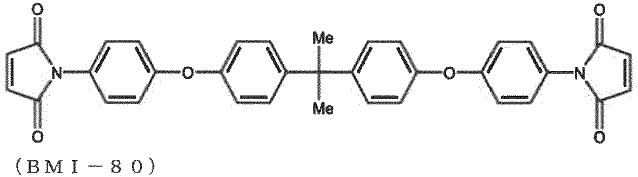

As a result of thermogravimetry, the amount of thermogravimetric weight loss at 400°C of the obtained film forming material for lithography was less than 10% (evaluation A). In addition, as a result of evaluation of solubility in PGMEA, the solubility was 5% by mass or more and less than 10% by mass (evaluation B), and the obtained film forming material for lithography was evaluated to have sufficient solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 7>

Five parts by mass of N,N'-biphenyl-based bismaleimide (BMI-70; manufactured by K·I Chemical Industry Co., LTD.) as a bismaleimide compound and 5 parts by mass of phenylmethane maleimide oligomer (BMI-2300; manufactured by Daiwa Kasei Industry Co., Ltd.) as an addition polymerization-type maleimide resin were compounded to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 8>

Ten parts by mass of BMI-70 as a bismaleimide compound and 0.1 part by mass of 2,4,5-triphenylimidazole (TPIZ) as a crosslinking promoting agent were compounded to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 5% by mass or more and less than 10% by mass (evaluation B), and the obtained film forming material for lithography was evaluated to have sufficient solubility.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 9>

Ten parts by mass of BMI-2300 was used as an addition polymerization-type maleimide resin. In addition, 0.1 part by mass of TPIZ was compounded as a crosslinking promoting agent to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the maleimide resin, 90 parts by mass of PGMEA as a solvent was added, and the resultant mixture was stirred with a stirrer for at least 3 hours or longer at room temperature to prepare a composition for film formation for lithography.

### <Example 10>

Ten parts by mass of MIR-3000-L was used as an addition polymerization-type maleimide resin. In addition, 0.1 part by mass of triphenylphosphine was compounded as a crosslinking promoting agent to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the maleimide resin, 90 parts by mass of PGMEA as a solvent was added, and the resultant mixture was stirred with a stirrer for at least 3 hours or longer at room temperature to prepare a composition for film formation for lithography.

### <Example 11>

Five parts by mass of BMI-70 as a bismaleimide compound and 5 parts by mass of BMI-2300 as an addition polymerization-type maleimide resin were used. In addition, 0.1 part by mass of TPIZ was compounded as a crosslinking promoting agent to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 12>

Ten parts by mass of BMI-50P as a bismaleimide compound and 0.1 part by mass of TPIZ as a crosslinking promoting agent were compounded to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 13>

Ten parts by mass of BMI-1000P as a bismaleimide compound and 0.1 part by mass of TPIZ as a crosslinking promoting agent were compounded to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 14>

Ten parts by mass of BMI-80 as a bismaleimide compound and 0.1 part by mass of TPIZ as a crosslinking promoting agent were compounded to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 15>

Five parts by mass of BMI-70 as a bismaleimide compound and 5 parts by mass of BMI-2300 as an addition polymerization-type maleimide resin were used. In addition, 2 parts by mass of benzoxazine (BF-BXZ; manufactured by KONISHI CHEMICAL IND. CO., LTD.) represented by the formula described below was used as a crosslinking agent and 0.1 part by mass of 2,4,5-triphenylimidazole (TPIZ) was compounded as a crosslinking promoting agent to prepare a film forming material for lithography.

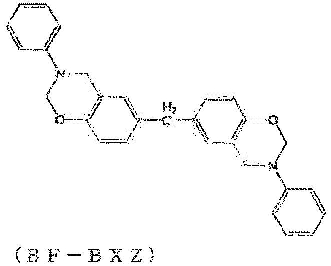

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 16>

Five parts by mass of BMI-70 as a bismaleimide compound and 5 parts by mass of BMI-2300 as an addition polymerization-type maleimide resin were used. In addition, 2 parts by mass of a biphenyl aralkyl-based epoxy resin (NC-3000-L; manufactured by Nippon Kayaku Co., Ltd.) represented by the formula described below was used as a crosslinking agent and 0.1 part by mass of TPIZ was compounded as a crosslinking promoting agent to prepare a film forming material for lithography.

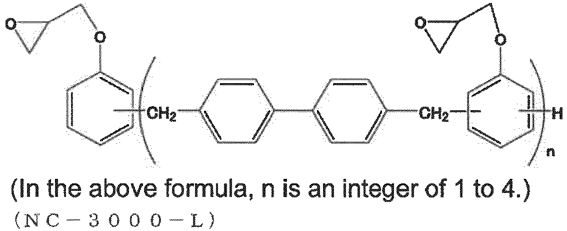

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 17>

Five parts by mass of BMI-70 as a bismaleimide compound and 5 parts by mass of BMI-2300 as an addition polymerization-type maleimide resin were used. In addition, 2 parts by mass of a diallylbisphenol A-based cyanate (DABPA-CN; manufactured by MITSUBISHI GAS CHEMICAL COMPANY, INC.) represented by the formula described below was used as a crosslinking agent and 0.1 part by mass of 2,4,5-triphenylimidazole (TPIZ) was compounded as a crosslinking promoting agent to prepare a film forming material for lithography.

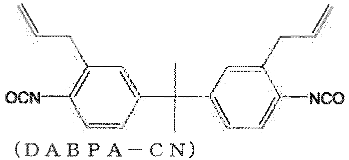

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 18>

Five parts by mass of BMI-70 as a bismaleimide compound and 5 parts by mass of BMI-2300 as an addition polymerization-type maleimide resin were used. In addition, 2 parts by mass of diallylbisphenol A (BPA-CA; manufactured by KONISHI CHEMICAL IND. CO., LTD.) represented by the formula described below was used as a crosslinking agent and 0.1 part by mass of 2,4,5-triphenylimidazole (TPIZ) was compounded as a crosslinking promoting agent to prepare a film forming material for lithography.

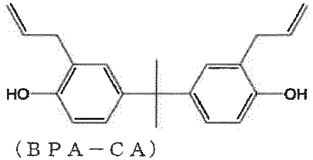

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 19>

Ten parts by mass of BMI-50P was used as a bismaleimide compound, 2 parts by mass of benzoxazine BF-BXZ represented by the formula described above was also used as a crosslinking agent, and 0.1 part by mass of TPIZ was compounded as a crosslinking promoting agent to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 20>

Ten parts by mass of BMI-1000P was used as a bismaleimide compound, 2 parts by mass of benzoxazine BF-BXZ represented by the formula described above was also used as a crosslinking agent, and 0.1 part by mass of TPIZ was compounded as a crosslinking promoting agent to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 21>

Ten parts by mass of BMI-80 was used as a bismaleimide compound, 2 parts by mass of benzoxazine BF-BXZ represented by the formula described above was also used as a crosslinking agent, and 0.1 part by mass of TPIZ was compounded as a crosslinking promoting agent to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 22>

Ten parts by mass of BMI-80 was used as a bismaleimide compound, 2 parts by mass of benzoxazine BF-BXZ represented by the formula described above was also used as a crosslinking agent, and in order to promote crosslinking, 0.1 part by mass of an acid generating agent, di-tertiary-butyldiphenyliodonium nonafluoromethanesulfonate (DTDPI; manufactured by Midori Kagaku Co., Ltd.) was compounded to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 23>

Five parts by mass of BMI-70 as a bismaleimide compound and 5 parts by mass of BMI-2300 as an addition polymerization-type maleimide resin were used. In addition, 2 parts by mass of benzoxazine BF-BXZ represented by the formula described above was used as a crosslinking agent to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 24>

Five parts by mass of BMI-70 as a bismaleimide compound and 5 parts by mass of BMI-2300 as an addition polymerization-type maleimide resin were used. In addition, 2 parts by mass of a biphenyl aralkyl-based epoxy resin (NC-3000-L; manufactured by Nippon Kayaku Co., Ltd.) represented by the formula described above was used as a crosslinking agent to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 25>

Five parts by mass of BMI-70 as a bismaleimide compound and 5 parts by mass of BMI-2300 as an addition polymerization-type maleimide resin were used. In addition, 2 parts by mass of a diallylbisphenol A-based cyanate (DABPA-CN; manufactured by MITSUBISHI GAS CHEMICAL COMPANY, INC.) represented by the formula described above was used as a crosslinking agent to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 26>

Five parts by mass of BMI-70 as a bismaleimide compound and 5 parts by mass of BMI-2300 as an addition polymerization-type maleimide resin were used. In addition, 2 parts by mass of a diallylbisphenol A (BPA-CA; manufactured by KONISHI CHEMICAL IND. CO., LTD.) represented by the formula described above was used as a crosslinking agent to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 27>

Ten parts by mass of BMI-50P was used as a bismaleimide compound and 2 parts by mass of benzoxazine BF-BXZ represented by the formula described above was also used as a crosslinking agent to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 28>

Ten parts by mass of BMI-1000P was used as a bismaleimide compound and 2 parts by mass of benzoxazine BF-BXZ represented by the formula described above was also used as a crosslinking agent to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 29>

Ten parts by mass of BMI-80 was used as a bismaleimide compound and 2 parts by mass of benzoxazine BF-BXZ represented by the formula described above was also used as a crosslinking agent to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 30>

Five parts by mass of BMI-70 as a bismaleimide compound and 5 parts by mass of BMI-2300 as an addition polymerization-type maleimide resin were used. In addition, 2 parts by mass of a diphenylmethane-based allylphenolic resin (APG-1; manufactured by Gun Ei Chemical Industry Co., Ltd.) represented by the formula described below was used as a crosslinking agent to prepare a film forming material for lithography.

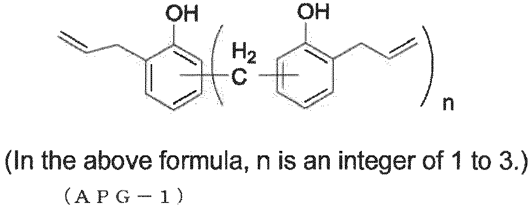

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 31>

Five parts by mass of BMI-70 as a bismaleimide compound and 5 parts by mass of BMI-2300 as an addition polymerization-type maleimide resin were used. In addition, 2 parts by mass of a diphenylmethane-based propenylphenolic resin (APG-2; manufactured by Gun Ei Chemical Industry Co., Ltd.) represented by the formula described below was used as a crosslinking agent to prepare a film forming material for lithography.

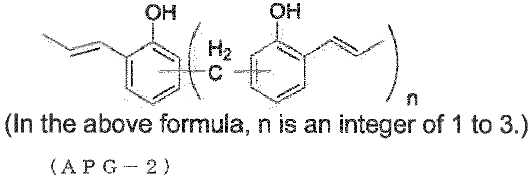

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 32>

Five parts by mass of BMI-70 as a bismaleimide compound and 5 parts by mass of BMI-2300 as an addition polymerization-type maleimide resin were used. In addition, 2 parts by mass of 4,4'-diaminodiphenylmethane (DDM; manufactured by Tokyo Chemical Industry Co., Ltd.) represented by the formula described below was used as a crosslinking agent to prepare a film forming material for lithography.

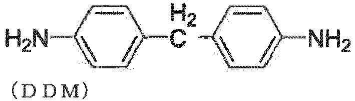

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have excellent solubility.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 33>

As a bismaleimide compound, 10 parts by mass of BMI-689 (manufactured by Designer Molecules Inc.) represented by the formula described below was used alone to prepare a film forming material for lithography.

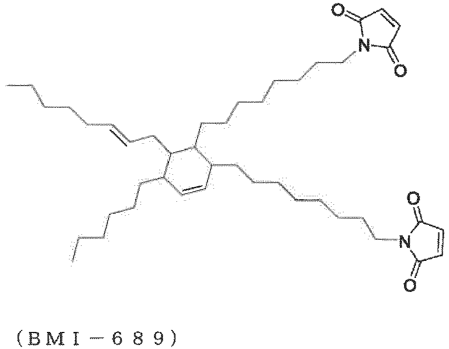

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have sufficient solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 34>

As a bismaleimide compound, 10 parts by mass of BMI-BY16-871 (manufactured by K·I Chemical Industry Co., LTD.) represented by the formula described below was used alone to prepare a film forming material for lithography.

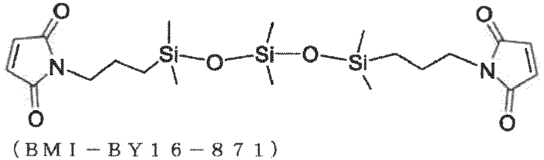

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have sufficient solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 35>

As a bismaleimide compound, 10 parts by mass of BMI-4,4'-BPE (manufactured by K·I Chemical Industry Co., LTD.) represented by the formula described below was used alone to prepare a film forming material for lithography.

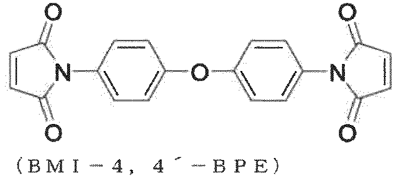

As a result of thermogravimetry, the amount of thermogravimetric weight loss at 400°C of the obtained film forming material for lithography was less than 10% (evaluation A). In addition, as a result of evaluation of solubility in PGMEA, the solubility was 5% by mass or more (evaluation B), and the obtained film forming material for lithography was evaluated to have sufficient solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 36>

As a bismaleimide compound, 10 parts by mass of BMI-3000J (manufactured by Designer Molecules Inc.) represented by the formula described below was used alone to prepare a film forming material for lithography.

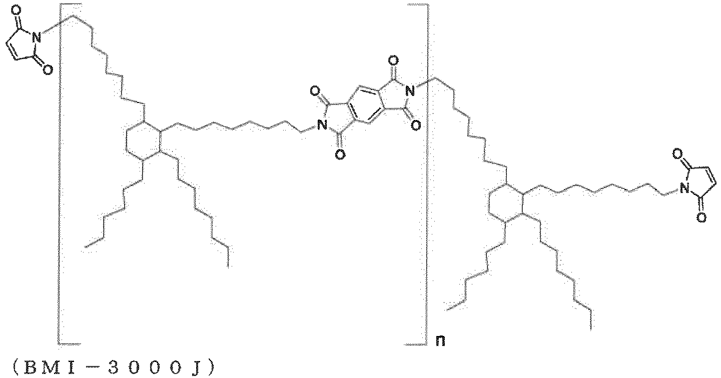

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have sufficient solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 37>

As a bismaleimide compound, 10 parts by mass of BMI-6000 (manufactured by Designer Molecules Inc.) represented by the formula described below was used alone to prepare a film forming material for lithography.

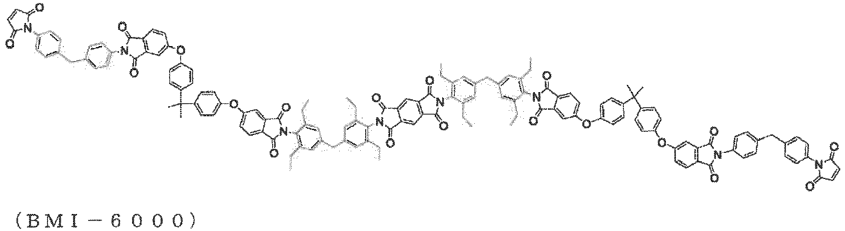

As a result of thermogravimetry, the amount of thermogravimetric weight loss at 400°C of the obtained film forming material for lithography was less than 10% (evaluation A). In addition, as a result of evaluation of solubility in PGMEA, the solubility was 5% by mass or more (evaluation B), and the obtained film forming material for lithography was evaluated to have solubility.

To 10 parts by mass of the film forming material for lithography, 90 parts by mass of CHN as a solvent was added, and the resultant mixture was stirred with a stirrer for at least 3 hours or longer at room temperature to prepare a composition for film formation for lithography.

### <Example 38>

As a bismaleimide compound, 10 parts by mass of 4-APCH maleimide obtained in Synthetic Working Example 1 was used alone to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have necessary solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 39>

As a bismaleimide compound, 10 parts by mass of TPE-R maleimide obtained in Synthetic Working Example 2 was used alone to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have necessary solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 40>

As a bismaleimide compound, 10 parts by mass of HFBAPP maleimide obtained in Synthetic Working Example 3 was used alone to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have necessary solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 41>

As a bismaleimide compound, 10 parts by mass of TFMB maleimide obtained in Synthetic Working Example 4 was used alone to prepare a film forming material for lithography.

As a result of thermogravimetry, the amount of thermogravimetric weight loss at 400°C of the obtained film forming material for lithography was less than 10% (evaluation A). In addition, as a result of evaluation of solubility in PGMEA, the solubility was 10% by mass or more (evaluation A), and the obtained film forming material for lithography was evaluated to have necessary solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 42>

As a bismaleimide compound, 10 parts by mass of DANPG maleimide obtained in Synthetic Working Example 5 was used alone to prepare a film forming material for lithography.

As a result of thermogravimetry, the amount of thermogravimetric weight loss at 400°C of the obtained film forming material for lithography was less than 20% (evaluation B). In addition, as a result of evaluation of solubility in PGMEA, the solubility was 10% by mass or more (evaluation A), and the obtained film forming material for lithography was evaluated to have necessary solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Example 43>

Ten parts by mass of BMI-70 was used as a bismaleimide compound and 0.1 part by mass of IRGACURE 184 (manufactured by BASF SE) represented by the formula described below was also compounded as a photo-radical polymerization initiator to prepare a film forming material for lithography.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

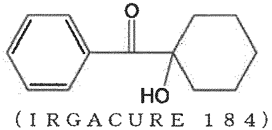

### <Example 44>

Ten parts by mass of BMI-2300 was used as an addition polymerization-type maleimide resin. In addition, 0.1 part by mass of IRGACURE 184 (manufactured by BASF SE) was compounded as a photo-radical polymerization initiator to prepare a film forming material for lithography.

To 10 parts by mass of the maleimide resin, 90 parts by mass of PGMEA as a solvent was added, and the resultant mixture was stirred with a stirrer for at least 3 hours or longer at room temperature to prepare a composition for film formation for lithography.

### <Example 45>

Ten parts by mass of MIR-3000-L was used as an addition polymerization-type maleimide resin. In addition, 0.1 part by mass of IRGACURE 184 (manufactured by BASF SE) was compounded as a photo-radical polymerization initiator to prepare a film forming material for lithography.

To 10 parts by mass of the maleimide resin, 90 parts by mass of PGMEA as a solvent was added, and the resultant mixture was stirred with a stirrer for at least 3 hours or longer at room temperature to prepare a composition for film formation for lithography.

### <Example 46>

Five parts by mass of BMI-70 as a bismaleimide compound and 5 parts by mass of BMI-2300 as an addition polymerization-type maleimide resin were used. In addition, 0.1 part by mass of IRGACURE 184 (manufactured by BASF SE) was compounded as a photo-radical polymerization initiator to prepare a film forming material for lithography.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 47>

Ten parts by mass of BMI-50P was used as a bismaleimide compound. In addition, 0.1 part by mass of IRGACURE 184 (manufactured by BASF SE) was compounded as a photo-radical polymerization initiator to prepare a film forming material for lithography.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 48>

Ten parts by mass of BMI-1000P was used as a bismaleimide compound and 0.1 part by mass of IRGACURE 184 (manufactured by BASF SE) represented by the formula described above was also compounded as a photo-radical polymerization initiator to prepare a film forming material for lithography.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 49>

Ten parts by mass of BMI-80 was used as a bismaleimide compound and 0.1 part by mass of IRGACURE 184 (manufactured by BASF SE) represented by the formula described above was also compounded as a photo-radical polymerization initiator to prepare a film forming material for lithography.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 50>

Five parts by mass of BMI-70 as a bismaleimide compound and 5 parts by mass of BMI-2300 as an addition polymerization-type maleimide resin were used. In addition, 2 parts by mass of BF-BXZ was used as a crosslinking agent and 0.1 part by mass of IRGACURE 184 (manufactured by BASF SE) was compounded as a photo-radical polymerization initiator to prepare a film forming material for lithography.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 51>

Five parts by mass of BMI-70 as a bismaleimide compound and 5 parts by mass of BMI-2300 as an addition polymerization-type maleimide resin were used. In addition, 2 parts by mass of NC-3000-L was used as a crosslinking agent and 0.1 part by mass of IRGACURE 184 (manufactured by BASF SE) was compounded as a photo-radical polymerization initiator to prepare a film forming material for lithography.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 52>

Five parts by mass of BMI-70 as a bismaleimide compound and 5 parts by mass of BMI-2300 as an addition polymerization-type maleimide resin were used. In addition, 2 parts by mass of DABPA-CN was used as a crosslinking agent and 0.1 part by mass of IRGACURE 184 (manufactured by BASF SE) was compounded as a photo-radical polymerization initiator to prepare a material for film formation for lithography.

To the total mass of 10 parts by mass of the bismaleimide compound and the addition polymerization-type maleimide resin in the material for film formation for lithography, 90 parts by mass of PGMEA as a solvent was added, and the resultant mixture was stirred with a stirrer for at least 3 hours or longer at room temperature to prepare a composition for film formation for lithography.

### <Example 53>

Five parts by mass of BMI-70 as a bismaleimide compound and 5 parts by mass of BMI-2300 as an addition polymerization-type maleimide resin were used. In addition, 2 parts by mass of BPA-CA was used as a crosslinking agent and 0.1 part by mass of IRGACURE 184 (manufactured by BASF SE) was compounded as a photo-radical polymerization initiator to prepare a film forming material for lithography.

To the total mass of 10 parts by mass of the bismaleimide compound and the addition polymerization-type maleimide resin in the film forming material for lithography, 90 parts by mass of PGMEA as a solvent was added, and the resultant mixture was stirred with a stirrer for at least 3 hours or longer at room temperature to prepare a composition for film formation for lithography.

### <Example 54>

Ten parts by mass of BMI-50P was used as a bismaleimide compound. In addition, 2 parts by mass of BF-BXZ was used as a crosslinking agent and 0.1 part by mass of IRGACURE 184 (manufactured by BASF SE) was compounded as a photo-radical polymerization initiator to prepare a film forming material for lithography.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 55>

Ten parts by mass of BMI-1000P was used as a bismaleimide compound. In addition, 2 parts by mass of BF-BXZ was used as a crosslinking agent and 0.1 part by mass of IRGACURE 184 (manufactured by BASF SE) was compounded as a photo-radical polymerization initiator to prepare a film forming material for lithography.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Example 56>

Ten parts by mass of BMI-80 was used as a bismaleimide compound. In addition, 2 parts by mass of BF-BXZ was used as a crosslinking agent and 0.1 part by mass of IRGACURE 184 (manufactured by BASF SE) was compounded as a photo-radical polymerization initiator to prepare a film forming material for lithography.

To 10 parts by mass of the bismaleimide compound, 90 parts by mass of PGMEA as a solvent was added, and the resultant mixture was stirred with a stirrer for at least 3 hours or longer at room temperature to prepare a composition for film formation for lithography.

### <Production Example 1>

A 4-neck flask (internal capacity: 10 L) equipped with a Dimroth condenser tube, a thermometer, and a stirring blade and having a detachable bottom was prepared. To this 4-neck flask, 1.09 kg (7 mol) of 1,5-dimethylnaphthalene (manufactured by Mitsubishi Gas Chemical Co., Inc.), 2.1 kg (28 mol as formaldehyde) of 40% by mass of an aqueous formalin solution (manufactured by Mitsubishi Gas Chemical Co., Inc.), and 0.97 ml of 98% by mass of sulfuric acid (manufactured by Kanto Chemical Co., Inc.) were fed in the current of nitrogen, and the mixture was reacted for 7 hours while being refluxed at 100°C at normal pressure. Subsequently, 1.8 kg of ethylbenzene (manufactured by Wako Pure Chemical Industries, Ltd., a special grade reagent) was added as a diluting solvent to the reaction solution, and the mixture was left to stand still, followed by removal of an aqueous phase as a lower phase. Neutralization and washing with water were further performed, and ethylbenzene and unreacted 1,5-dimethylnaphthalene were distilled off under reduced pressure to obtain 1.25 kg of a dimethylnaphthalene formaldehyde resin as a light brown solid.

The molecular weight of the obtained dimethylnaphthalene formaldehyde resin was as follows: number average molecular weight (Mn): 562, weight average molecular weight (Mw): 1168, and dispersity (Mw/Mn): 2.08.

Then, a 4-neck flask (internal capacity: 0.5 L) equipped with a Dimroth condenser tube, a thermometer, and a stirring blade was prepared. To this 4-necked flask, 100 g (0.51 mol) of the dimethylnaphthalene formaldehyde resin obtained as mentioned above, and 0.05 g of p-toluenesulfonic acid were added in a nitrogen stream, and the temperature was raised to 190°C at which the mixture was then heated for 2 hours, followed by stirring. Subsequently, 52.0 g (0.36 mol) of 1-naphthol was further added thereto, the temperature was further elevated to 220°C, and the mixture was reacted for 2 hours. After dilution with a solvent, neutralization and washing with water were performed, and the solvent was distilled off under reduced pressure to obtain 126.1 g of a modified resin (CR-1) as a black-brown solid.

The obtained resin (CR-1) had Mn: 885, Mw: 2220, and Mw/Mn: 4.17.

As a result of thermogravimetry (TG), the amount of thermogravimetric weight loss at 400°C of the obtained resin was greater than 25% (evaluation C). Therefore, application to high temperature baking was evaluated to be difficult.

As a result of evaluation of solubility in PGMEA, the solubility was 10% by mass or more (evaluation A), and the obtained resin was evaluated to have excellent solubility.

Note that the above-described Mn, Mw and Mw/Mn were measured by carrying out gel permeation chromatography (GPC) analysis under the following conditions to determine the molecular weight in terms of polystyrene.
Apparatus: Shodex GPC-101 model (manufactured by SHOWA DENKO K.K.)
Column: KF-80M x 3
Eluent: THF 1 mL/min
Temperature: 40°C

### <Example 57>

To 8 parts by mass of a phenol compound (BisN-1) represented by the formula described below, described in International Publication No. WO 2013/024779, 2 parts by mass of BMI-80 was added as a bismaleimide compound to prepare a film forming material for lithography.

As a result of thermogravimetry, the amount of thermogravimetric weight loss at 400°C of the obtained film forming material for lithography was less than 5%. In addition, as a result of evaluation of solubility in PGMEA, the solubility was 10% by mass or more (evaluation A), and the obtained film forming material for lithography was evaluated to have necessary solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

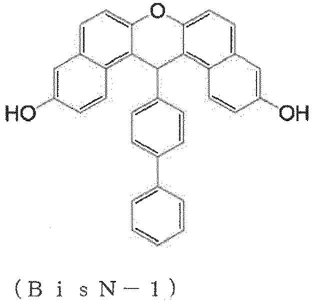

### <Example 58>

To 8 parts by mass of the phenol compound (BisN-1) represented by the formula described above, 2 parts by mass of BMI-2300 was added as a bismaleimide compound to prepare a film forming material for lithography.

As a result of thermogravimetry, the amount of thermogravimetric weight loss at 400°C of the obtained film forming material for lithography was less than 5%. In addition, as a result of evaluation of solubility in PGMEA, the solubility was 10% by mass or more (evaluation A), and the obtained film forming material for lithography was evaluated to have necessary solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Reference Example 1>

Ten parts by mass of the phenol compound (BisN-1) represented by the formula described above was used alone to prepare a film forming material for lithography.

As a result of thermogravimetry, the amount of thermogravimetric weight loss at 400°C of the obtained film forming material for lithography was less than 10%. In addition, as a result of evaluation of solubility in PGMEA, the solubility was 10% by mass or more (evaluation A), and the obtained film forming material for lithography was evaluated to have necessary solubility.

To 10 parts by mass of the film forming material for lithography, 90 parts by mass of PGMEA as a solvent was added, and the resultant mixture was stirred with a stirrer for at least 3 hours or longer at room temperature to prepare a composition for film formation for lithography.

### <Examples 1 to 42 and Comparative Examples 1 to 2>

Each composition for film formation for lithography corresponding to Examples 1 to 42 and Comparative Examples 1 to 2 was prepared using film forming materials for lithography obtained in the above Examples 1 to 42 and the resin obtained in the above Production Example 1 according to the composition shown in Table 1. Then, a silicon supporting material was spin coated with each of these compositions for film formation for lithography of Examples 1 to 42 and Comparative Examples 1 to 2, and then baked at 240°C for 60 seconds and further at 400°C for 120 seconds to prepare each underlayer film with a film thickness of 200 nm. From the difference in film thickness before and after the baking at 400°C, the decreasing rate of film thickness (%) was calculated to evaluate the film heat resistance of each underlayer film. Then, the etching resistance was evaluated under the conditions shown below.

In addition, the embedding properties to a supporting material having difference in level and the film flatness were evaluated under the conditions shown below.

### <Examples 43 to 56>

Each composition for film formation for lithography corresponding to the above Examples 43 to 56 was prepared according to the composition shown in Table 2. Then, a silicon supporting material was spin coated with each of these compositions for film formation for lithography of Examples 43 to 56, and then baked at 110°C for 60 seconds to remove the solvent in the coated film. Subsequently, the film was cured using a high pressure mercury lamp with an accumulated light exposure of 600 mJ/cm² and an irradiation time of 20 seconds, and further baked at 400°C for 120 seconds to prepare each underlayer film with a film thickness of 200 nm. From the difference in film thickness before and after the baking at 400°C, the decreasing rate of film thickness (%) was calculated to evaluate the film heat resistance of each underlayer film. Then, the etching resistance was evaluated under the conditions shown below.

In addition, the embedding properties to a supporting material having difference in level and the film flatness were evaluated under the conditions shown below.

### <Examples 57 and 58 and Reference Example 1>

Each composition for film formation for lithography corresponding to the above Examples 57 and 58 and Reference Example 1 was prepared according to the composition shown in Table 3. Then, a silicon supporting material was spin coated with each of these compositions for film formation for lithography of Examples 57 and 58 and Reference Example 1, and then baked at 110°C for 60 seconds to remove the solvent in the coated film. Subsequently, the film was cured using a high pressure mercury lamp with an accumulated light exposure of 600 mJ/cm² and an irradiation time of 20 seconds, and further baked at 400°C for 120 seconds to prepare each underlayer film with a film thickness of 200 nm. From the difference in film thickness before and after the baking at 400°C, the decreasing rate of film thickness (%) was calculated to evaluate the film heat resistance of each underlayer film. Then, the etching resistance was evaluated under the conditions shown below.

In addition, the embedding properties to a supporting material having difference in level and the film flatness were evaluated under the conditions shown below.

### [Evaluation of film heat resistance]

### <Evaluation criteria>

S: Decreasing rate of film thickness before and after baking at 400°C ≤ 10%
A: Decreasing rate of film thickness before and after baking at 400°C ≤ 15%
B: Decreasing rate of film thickness before and after baking at 400°C ≤ 20%
C: Decreasing rate of film thickness before and after baking at 400°C > 20%

### [Etching test]

Etching apparatus: RIE-10NR manufactured by Samco International, Inc.
Output: 50 W
Pressure: 4 Pa
Time: 2 min
Etching gas
CF₄ gas flow rate: O2 gas flow rate = 5:15 (sccm)

### [Evaluation of etching resistance]

The evaluation of etching resistance was conducted by the following procedures.

First, an underlayer film of novolac was prepared under the same conditions as Example 1 except that novolac (PSM 4357 manufactured by Gun Ei Chemical Industry Co., Ltd.) was used instead of the film forming material for lithography in Example 1 and the drying temperature was 110°C. Then, this underlayer film of novolac was subjected to the etching test mentioned above, and the etching rate was measured.

Next, underlayer films of Examples 1 to 58, Comparative Examples 1 and 2, and Reference Example 1 were subjected to the etching test described above in the same way as above, and the etching rate was measured.

Then, the etching resistance was evaluated according to the following evaluation criteria on the basis of the etching rate of the underlayer film of novolac. From a practical viewpoint, evaluation S described below is particularly preferable, and evaluation A and evaluation B are preferable.

### <Evaluation criteria>

S: The etching rate was less than -30% as compared with the underlayer film of novolac.
A: The etching rate was -30% or more to less than -20% as compared with the underlayer film of novolac.
B: The etching rate was -20% or more to less than -10% as compared with the underlayer film of novolac.
C: The etching rate was -10% or more and 0% or less as compared with the underlayer film of novolac.

### [Evaluation of embedding properties to supporting material having difference in level]

The embedding properties to a supporting material having difference in level were evaluated by the following procedures.

A SiO₂ supporting material having a film thickness of 80 nm and a line and space pattern of 60 nm was coated with a composition for underlayer film formation for lithography, and baked at 240°C for 60 seconds to form a 90 nm underlayer film. The cross section of the obtained film was cut out and observed under an electron microscope to evaluate the embedding properties to a supporting material having difference in level.

### <Evaluation criteria>

A: The underlayer film was embedded without defects in the asperities of the SiO₂ supporting material having a line and space pattern of 60 nm.
C: The asperities of the SiO₂ supporting material having a line and space pattern of 60 nm had defects which hindered the embedding of the underlayer film.

### [Evaluation of flatness]

Onto a SiO₂ supporting material having difference in level on which trenches with a width of 100 nm, a pitch of 150 nm and a depth of 150 nm (aspect ratio: 1.5) and trenches with a width of 5 µm and a depth of 180 nm (open space) were mixedly present, each of the obtained compositions for film formation was coated.
Subsequently, it was calcined at 240°C for 120 seconds under the air atmosphere to form a resist underlayer film having a film thickness of 200 nm. The shape of this resist underlayer film was observed with a scanning electron microscope ("S-4800" from Hitachi High-Technologies Corporation), and the difference between the maximum value and the minimum value of the film thickness of the resist underlayer film on the trench or space (ΔFT) was measured.

### <Evaluation criteria>

S: ΔFT < 10 nm (best flatness)
A: 10 nm ≤ ΔFT < 20 nm (good flatness)
B: 20 nm ≤ ΔFT < 40 nm (partially good flatness)
C: 40 nm ≤ ΔFT (poor flatness)

### [Table 1]

**Table 1**

| | Bismaleimide and/or maleimide resin | Crosslinking agent | Crosslinking Promoting agent | Solvent | Film heat resistance | Etching resistance | Embedding properties | Flatness |
|---|---|---|---|---|---|---|---|---|
| Example 1 | BMI-70 (10) | - | - | PGMEA (90) | A | A | A | A |
| Example 2 | BMI-2300 (10) | - | - | PGMEA (90) | A | A | A | B |
| Example 3 | MIR-3000-L (10) | - | - | PGMEA (90) | A | A | A | B |
| Example 4 | BMI-70 (5) | - | - | PGMEA (90) | A | A | A | A |
| | BMI-2300 (5) | | | | | | | |
| Example 5 | BMI-50P(10) | - | - | PGMEA (90) | A | A | A | A |
| Example 6 | BMI-1000P(10) | - | - | PGMEA (90) | B | - | A | A |
| Example 7 | BMI-80(10) | - | - | PGMEA (90) | A | A | A | A |
| Example 8 | BMI-70 (10) | - | TPIZ (0.1) | PGMEA (90) | S | S | A | A |
| Example 9 | BMI-2300 (10) | - | TPIZ (0.1) | PGMEA (90) | S | S | A | B |
| Example 10 | MIR-3000-L (10) | - | TPIZ (0.1) | PGMEA (90) | S | S | A | B |
| Example 11 | BMI-70 (5) | - | TPIZ (0.1) | PGMEA (90) | S | S | A | A |
| | BMI-2300 (5) | | | | | | | |
| Example 12 | BMI-50P(10) | - | TPIZ (0.1) | PGMEA (90) | S | S | A | A |
| Example 13 | BMI-1000P(10) | - | TPIZ (0.1) | PGMEA (90) | B | - | A | A |
| Example 14 | BMI-80(10) | - | TPIZ (0.1) | PGMEA (90) | S | S | A | A |
| Example 15 | BMI-70 (5) | BF-BXZ(2) | TPIZ (0.1) | PGMEA (90) | S | A | A | A |
| | BMI-2300 (5) | | | | | | | |
| Example 16 | BMI-70 (5) | NC-3000-L(2) | TPIZ (0.1) | PGMEA (90) | S | B | A | A |
| | BMI-2300 (5) | | | | | | | |
| Example 17 | BMI-70 (5) | DABPA-CN(2) | TPIZ (0.1) | PGMEA (90) | S | B | A | A |
| | BMI-2300 (5) | | | | | | | |
| Example 18 | BMI-70 (5) | BPA-CA(2) | TPIZ (0.1) | PGMEA (90) | S | B | A | A |
| | BMI-2300 (5) | | | | | | | |
| Example 19 | BMI-50P(10) | BF-BXZ(2) | TPIZ (0.1) | PGMEA (90) | S | S | A | A |
| Example 20 | BMI-1000P(10) | BF-BXZ(2) | TPIZ (0.1) | PGMEA (90) | B | - | A | A |
| Example 21 | BMI-80(10) | BF-BXZ(2) | TPIZ (0.1) | PGMEA (90) | S | A | A | A |
| Example 22 | BMI-80(10) | BF-BXZ(2) | DTDPI(0.1) | PGMEA (90) | S | A | A | A |
| Example 23 | BMI-70 (5) | BF-BXZ(2) | - | PGMEA (90) | A | A | A | A |
| | BMI-2300 (5) | | | | | | | |
| Example 24 | BMI-70 (5) | NC-3000-L(2) | - | PGMEA (90) | A | B | A | A |
| | BMI-2300 (5) | | | | | | | |
| Example 25 | BMI-70 (5) | DABPA-CN(2) | - | PGMEA (90) | A | B | A | A |
| | BMI-2300 (5) | | | | | | | |
| Example 26 | BMI-70 (5) | BPA-CA(2) | - | PGMEA (90) | A | B | A | A |
| | BMI-2300 (5) | | | | | | | |
| Example 27 | BMI-50P(10) | BF-BXZ(2) | - | PGMEA (90) | A | S | A | A |
| Example 28 | BMI-1000P(10) | BF-BXZ(2) | - | PGMEA (90) | B | - | A | A |
| Example 29 | BMI-80(10) | BF-BXZ(2) | - | PGMEA (90) | A | S | A | A |
| Example 30 | BMI-70 (5) | APG-1(2) | - | PGMEA (90) | A | A | A | A |
| | BMI-2300 (5) | | | | | | | |
| Example 31 | BMI-70 (5) | APG-2(2) | - | PGMEA (90) | S | A | A | A |
| | BMI-2300 (5) | | | | | | | |
| Example 32 | BMI-70 (5) | DDM(2) | - | PGMEA (90) | A | A | A | A |
| | BMI-2300 (5) | | | | | | | |
| Example 33 | BMI-689(10) | - | - | PGMEA (90) | A | A | A | A |
| Example 34 | BMI-BY16-871(10) | - | - | PGMEA (90) | B | A | A | A |
| Example 35 | BMI-4,4'-BPE(10) | - | - | PGMEA (90) | A | S | A | A |
| Example 36 | BMI-3000J(10) | - | - | PGMEA (90) | A | A | A | A |
| Example 37 | BMI-6000(10) | - | - | PGMEA (90) | A | S | A | A |
| Example 38 | 4-APAH maleimide (10) | - | - | PGMEA (90) | A | A | A | A |
| Example 39 | TPE-R maleimide (10) | - | - | PGMEA (90) | A | A | A | A |
| Example 40 | HFBAPP maleimide (10) | - | - | PGMEA (90) | A | B | A | A |
| Example 41 | TFMB maleimide (10) | - | - | PGMEA (90) | A | B | A | A |
| Example 42 | DANPG maleimide (10) | - | - | PGMEA (90) | B | A | A | A |
| Comparative Example 1 | CR-1(10) | NC-3000-L(4) | TPIZ (0.1) | PGMEA (90) | C | C | C | C |
| Comparative Example 2 | CR-1(10) | - | - | PGMEA (90) | C | C | C | C |

| | | | | | | | | |
|---|---|---|---|---|---|---|---|---|
| * Parts by mass of each component are shown in parentheses. | | | | | | | | |

### [Table 2]

**Table 2**

| | Bismaleimide and/or maleimide resin | Crosslinking agent | Radical polymerization initiator | Solvent | Film heat resistance | Etching resistance | Embedding properties | Flatness |
|---|---|---|---|---|---|---|---|---|
| Example 43 | BMI-70 (10) | - | IRGACURE 184 (0.1) | PGMEA (90) | A | S | A | A |
| Example 44 | BMI-2300 (10) | - | IRGACURE 184 (0.1) | PGMEA (90) | A | S | A | A |
| Example 45 | MIR-3000-L (10) | - | IRGACURE 184 (0.1) | PGMEA (90) | A | S | A | A |
| Example 46 | BMI-70 (5) | - | IRGACURE 184 (0.1) | PGMEA (90) | A | S | A | A |
| | BMI-2300 (5) | | | | | | | |
| Example 47 | BMI-50P(10) | - | IRGACURE 184 (0.1) | PGMEA (90) | A | S | A | A |
| Example 48 | BMI-1000P(10) | - | IRGACURE 184 (0.1) | PGMEA (90) | B | - | A | A |
| Example 49 | BMI-80(10) | - | IRGACURE 184 (0.1) | PGMEA (90) | A | S | A | A |
| Example 50 | BMI-70 (5) | BF-BXZ(2) | IRGACURE 184 (0.1) | PGMEA (90) | S | A | A | A |
| | BMI-2300 (5) | | | | | | | |
| Example 51 | BMI-70 (5) | NC-3000-L(2) | IRGACURE 184 (0.1) | PGMEA (90) | S | B | A | A |
| | BMI-2300 (5) | | | | | | | |
| Example 52 | BMI-70 (5) | DABPA-CN(2) | IRGACURE 184 (0.1) | PGMEA (90) | S | B | A | A |
| | BMI-2300 (5) | | | | | | | |
| Example 53 | BMI-70 (5) | BPA-CA(2) | IRGACURE 184 (0.1) | PGMEA (90) | S | B | A | A |
| | BMI-2300 (5) | | | | | | | |
| Example 54 | BMI-50P(10) | BF-BXZ(2) | IRGACURE 184 (0.1) | PGMEA (90) | S | A | A | A |
| Example 55 | BMI-1000P(10) | BF-BXZ(2) | IRGACURE 184 (0.1) | PGMEA (90) | B | - | A | A |
| Example 56 | BMI-80(10) | BF-BXZ(2) | IRGACURE 184 (0.1) | PGMEA (90) | S | B | A | A |

| | | | | | | | | |
|---|---|---|---|---|---|---|---|---|
| * Parts by mass of each component are shown in parentheses. | | | | | | | | |

### [Table 3]

**Table 3**

| | Main agent | Bismaleimide and/or maleimide resin | Solvent | Film heat resistance | Etching resistance | Embedding properties | Flatness |
|---|---|---|---|---|---|---|---|
| Example 57 | BisN-1(8) | BMI-80(2) | PGMEA (90) | A | A | A | A |
| Example 58 | BisN-1(8) | BMI-2300(2) | PGMEA (90) | A | A | A | A |
| Reference Example 1 | BisN-1(10) | - | PGMEA (90) | B | A | A | A |

| | | | | | | | |
|---|---|---|---|---|---|---|---|
| * Parts by mass of each component are shown in parentheses. | | | | | | | |

### <Example 59>

A SiO₂ supporting material with a film thickness of 300 nm was coated with the composition for film formation for lithography in Example 8, and baked at 240°C for 60 seconds and further at 400°C for 120 seconds to form an underlayer film with a film thickness of 70 nm. This underlayer film was coated with a resist solution for ArF and baked at 130°C for 60 seconds to form a photoresist layer with a film thickness of 140 nm. The resist solution for ArF used was prepared by compounding 5 parts by mass of a compound of the following formula (22), 1 part by mass of triphenylsulfonium nonafluoromethanesulfonate, 2 parts by mass of tributylamine, and 92 parts by mass of PGMEA.

Note that the compound of the following formula (22) was prepared as follows. 4.15 g of 2-methyl-2-methacryloyloxyadamantane, 3.00 g of methacryloyloxy-γ-butyrolactone, 2.08 g of 3-hydroxy-1-adamantyl methacrylate, and 0.38 g of azobisisobutyronitrile were dissolved in 80 mL of tetrahydrofuran to prepare a reaction solution. This reaction solution was polymerized for 22 hours with the reaction temperature kept at 63°C in a nitrogen atmosphere. Then, the reaction solution was added dropwise into 400 mL of n-hexane. The product resin thus obtained was solidified and purified, and the resulting white powder was filtered and dried overnight at 40°C under reduced pressure to obtain a compound represented by the following formula.

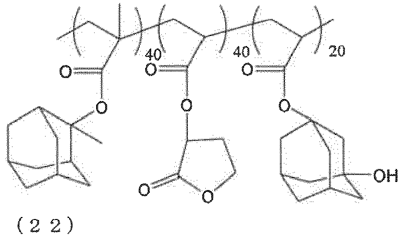

In the above formula (22), 40, 40 and 20 represent the ratio of each constituent unit and do not represent a block copolymer.

Subsequently, the photoresist layer was exposed using an electron beam lithography system (manufactured by ELIONIX INC.; ELS-7500, 50 keV), baked (PEB) at 115°C for 90 seconds, and developed for 60 seconds in 2.38% by mass tetramethylammonium hydroxide (TMAH) aqueous solution to obtain a positive type resist pattern. The evaluation results are shown in Table 3.

### <Example 60>

A positive type resist pattern was obtained in the same way as Example 59 except that the composition for underlayer film formation for lithography in Example 9 was used instead of the composition for underlayer film formation for lithography in the above Example 8. The evaluation results are shown in Table 4.

### <Example 61>

A positive type resist pattern was obtained in the same way as Example 59 except that the composition for underlayer film formation for lithography in Example 10 was used instead of the composition for underlayer film formation for lithography in the above Example 8. The evaluation results are shown in Table 4.

### <Example 62>

A positive type resist pattern was obtained in the same way as Example 59 except that the composition for underlayer film formation for lithography in Example 11 was used instead of the composition for underlayer film formation for lithography in the above Example 8. The evaluation results are shown in Table 4.

### <Comparative Example 3>

The same operations as in Example 59 were performed except that no underlayer film was formed so that a photoresist layer was formed directly on a SiO₂ supporting material to obtain a positive type resist pattern. The evaluation results are shown in Table 4.

### [Evaluation]

Concerning each of Examples 59 to 62 and Comparative Example 3, the shapes of the obtained 55 nm L/S (1:1) and 80 nm L/S (1:1) resist patterns were observed under an electron microscope (S-4800) manufactured by Hitachi, Ltd. The shapes of the resist patterns after development were evaluated as goodness when having good rectangularity without pattern collapse, and as poorness if this was not the case. The smallest line width having good rectangularity without pattern collapse as a result of this observation was used as an index for resolution evaluation. The smallest electron beam energy quantity capable of lithographing good pattern shapes was used as an index for sensitivity evaluation.

### [Table 4]

**Table 4**

| | Composition for film formation for lithography | Resolution (nmL/S) | Sensitivity (µC/cm²) | Resist pattern shape after development |
|---|---|---|---|---|
| Example 59 | That described in Example 8 | 55 | 16 | Good |
| Example 60 | That described in Example 9 | 60 | 15 | Good |
| Example 61 | That described in Example 10 | 53 | 15 | Good |
| Example 62 | That described in Example 11 | 50 | 16 | Good |
| Comparative Example 3 | None | 90 | 42 | Poor |

As is evident from Table 4, Examples 59 to 62 using the composition for film formation for lithography of the present embodiment including a bismaleimide compound and/or an addition polymerization-type maleimide resin were confirmed to be significantly superior in both resolution and sensitivity to Comparative Example 3. Also, the resist pattern shapes after development were confirmed to have good rectangularity without pattern collapse. Furthermore, the difference in the resist pattern shapes after development indicated that the underlayer films of Examples 59 to 62 obtained from the compositions for film formation for lithography of Examples 8 to 11 have good adhesiveness to a resist material.

The film forming material for lithography of the present embodiment has relatively high heat resistance, relatively high solvent solubility, excellent embedding properties to a supporting material having difference in level and film flatness, and is applicable to a wet process. Therefore, the composition for film formation for lithography comprising the film forming material for lithography can be utilized widely and effectively in various applications that require such performances. In particular, the present invention can be utilized particularly effectively in the field of underlayer films for lithography and underlayer films for multilayer resist.

## Claims

1. A film forming material for lithography comprising a compound having a group of the following formula (0): 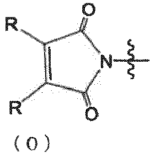 wherein each R is independently selected from the group consisting of a hydrogen atom and an alkyl group having 1 to 4 carbon atoms.

2. The film forming material for lithography according to claim 1, wherein the compound having a group of the above formula (0) is at least one selected from the group consisting of a polymaleimide compound and a maleimide resin.

3. The film forming material for lithography according to claim 1 or 2, wherein the compound having a group of the above formula (0) is at least one selected from the group consisting of a bismaleimide compound and an addition polymerization-type maleimide resin.

4. The film forming material for lithography according to claim 3, wherein the bismaleimide compound is represented by the following formula (1): 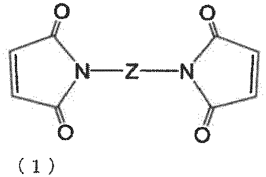 wherein Z is a divalent hydrocarbon group having 1 to 100 carbon atoms and optionally having a heteroatom.

5. The film forming material for lithography according to claim 3 or 4, wherein the bismaleimide compound is represented by the following formula (1A): 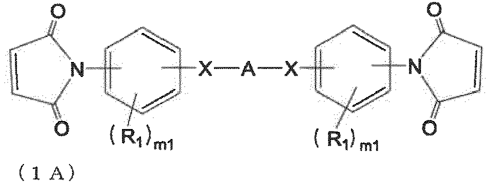 wherein
each X is independently a single bond, -O-, -CH₂-, - C(CH₃)₂-, -CO-, -C(CF₃)₂-, -CONH- or -COO-;
A is a single bond, an oxygen atom or a divalent hydrocarbon group having 1 to 80 carbon atoms and optionally having a heteroatom;
each R₁ is independently a group having 0 to 30 carbon atoms and optionally having a heteroatom; and
each m1 is independently an integer of 0 to 4.

6. The film forming material for lithography according to any one of claims 3 to 5, wherein the bismaleimide compound is represented by the following formula (1A): 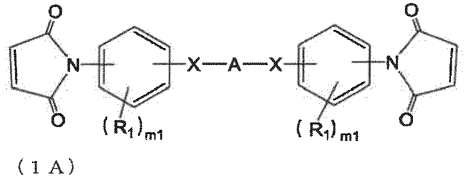 wherein
each X is independently a single bond, -O-, -CH₂-, - C(CH₃)₂-, -CO-, -C(CF₃)₂-, -CONH- or -COO-;
A is a single bond, an oxygen atom, -(CH₂)ₙ-, - CH₂C(CH₃)₂CH₂-, -(C(CH₃)₂)ₙ-, -(O(CH₂)ₘ₂)ₙ-, - (O(C₆H₄))ₙ- or any of the following structures: 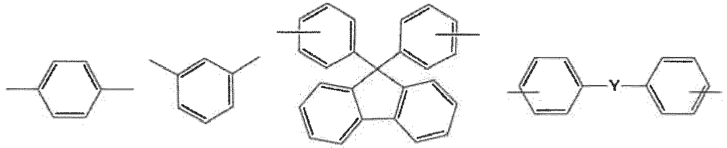 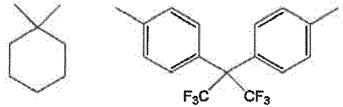
Y is a single bond, -O-, -CH₂-, -C(CH₃)₂-, -C(CF₃)₂-, 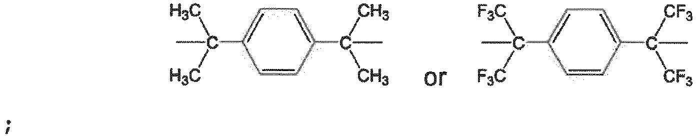
each R₁ is independently a group having 0 to 30 carbon atoms and optionally having a heteroatom; and
n is an integer of 0 to 20; and
m1 and m2 are each independently an integer of 0 to 4.

7. The film forming material for lithography according to any one of claims 3 to 6, wherein the addition polymerization-type maleimide resin is represented by the following formula (2): 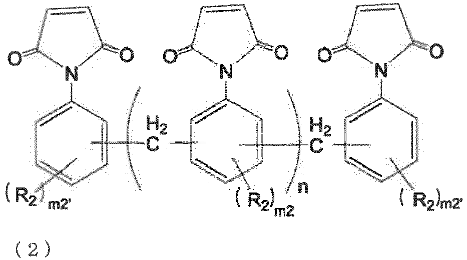 wherein
each R₂ is independently a group having 0 to 10 carbon atoms and optionally having a heteroatom;
each m2 is independently an integer of 0 to 3;
each m2' is independently an integer of 0 to 4; and
n is an integer of 1 to 4,
or the following formula (3): 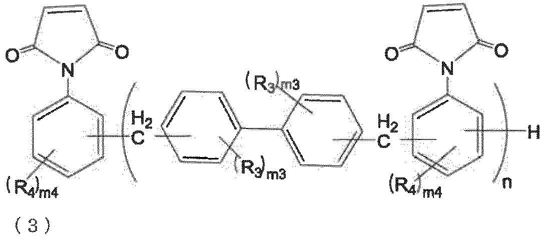 wherein
R₃ and R₄ are each independently a group having 0 to 10 carbon atoms and optionally having a heteroatom;
each m3 is independently an integer of 0 to 4;
each m4 is independently an integer of 0 to 4; and
n is an integer of 1 to 4.

8. The film forming material for lithography according to any one of claims 1 to 7, further comprising a crosslinking agent.

9. The film forming material for lithography according to claim 8, wherein the crosslinking agent is at least one selected from the group consisting of a phenol compound, an epoxy compound, a cyanate compound, an amino compound, a benzoxazine compound, a melamine compound, a guanamine compound, a glycoluril compound, a urea compound, an isocyanate compound and an azide compound.

10. The film forming material for lithography according to claim 8 or 9, wherein the crosslinking agent has at least one allyl group.

11. The film forming material for lithography according to any one of claims 8 to 10, wherein a content ratio of the crosslinking agent is 0.1 to 100 parts by mass based on 100 parts by mass of a total mass of the bismaleimide compound and the addition polymerization-type maleimide resin.

12. The film forming material for lithography according to any one of claims 1 to 11, further comprising a crosslinking promoting agent.

13. The film forming material for lithography according to claim 12, wherein the crosslinking promoting agent is at least one selected from the group consisting of an amine, an imidazole, an organic phosphine and a Lewis acid.

14. The film forming material for lithography according to claim 12 or 13, wherein a content ratio of the crosslinking promoting agent is 0.1 to 5 parts by mass based on 100 parts by mass of a total mass of the bismaleimide compound and the addition polymerization-type maleimide resin.

15. The film forming material for lithography according to any one of claims 1 to 14, further comprising a radical polymerization initiator.

16. The film forming material for lithography according to claim 15, wherein the radical polymerization initiator is at least one selected from the group consisting of a ketone-based photopolymerization initiator, an organic peroxide-based polymerization initiator and an azo-based polymerization initiator.

17. The film forming material for lithography according to claim 15 or 16, wherein a content ratio of the radical polymerization initiator is 0.05 to 25 parts by mass based on 100 parts by mass of a total mass of the bismaleimide compound and the addition polymerization-type maleimide resin.

18. A composition for film formation for lithography comprising the film forming material for lithography according to any one of claims 1 to 17 and a solvent.

19. The composition for film formation for lithography according to claim 18, further comprising an acid generating agent.

20. The composition for film formation for lithography according to claim 18 or 19, wherein the film for lithography is an underlayer film for lithography.

21. An underlayer film for lithography formed by using the composition for film formation for lithography according to claim 20.

22. A method for forming a resist pattern, comprising the steps of:
forming an underlayer film on a supporting material by using the composition for film formation for lithography according to claim 20;
forming at least one photoresist layer on the underlayer film; and
irradiating a predetermined region of the photoresist layer with radiation for development.

23. A method for forming a circuit pattern, comprising the steps of:
forming an underlayer film on a supporting material by using the composition for film formation for lithography according to claim 20;
forming an intermediate layer film on the underlayer film by using a resist intermediate layer film material having a silicon atom;
forming at least one photoresist layer on the intermediate layer film;
irradiating a predetermined region of the photoresist layer with radiation for development, thereby forming a resist pattern;
etching the intermediate layer film with the resist pattern as a mask;
etching the underlayer film with the obtained intermediate layer film pattern as an etching mask; and
etching the supporting material with the obtained underlayer film pattern as an etching mask, thereby forming a pattern on the supporting material.

24. A purification method comprising the steps of:
obtaining an organic phase by dissolving the film forming material for lithography according to any one of claims 1 to 17 in a solvent; and
extracting impurities in the film forming material for lithography by bringing the organic phase into contact with an acidic aqueous solution (a first extraction step), wherein
the solvent used in the step of obtaining the organic phase comprises a solvent that is incompatible with water.

25. The purification method according to claim 24, wherein
the acidic aqueous solution is an aqueous mineral acid solution or an aqueous organic acid solution;
the aqueous mineral acid solution comprises one or more selected from the group consisting of hydrochloric acid, sulfuric acid, nitric acid and phosphoric acid; and
the aqueous organic acid solution comprises one or more selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid and trifluoroacetic acid.

26. The purification method according to claim 24 or 25, wherein the solvent that is incompatible with water is one or more solvents selected from the group consisting of toluene, 2-heptanone, cyclohexanone, cyclopentanone, methyl isobutyl ketone, propylene glycol monomethyl ether acetate and ethyl acetate.

27. The purification method according to any one of claims 24 to 26, further comprising the step of extracting impurities in the film forming material for lithography by bringing the organic phase into contact with water after the first extraction step (a second extraction step).
